# EUROPÄISCHE PATENTSCHRIFT

(11) **EP 4 197 586 B1**
(45) Veröffentlichungstag und Bekanntmachung des Hinweises auf die Patenterteilung: **29.01.2025**
(21) Anmeldenummer: 21216173.1
(22) Anmeldetag: 20.12.2021
(51) Int. Cl.: A61N 1/04, A61N 1/36

(54) **PERSONALISIERTES ELEKTRODEN-INTERFACE ZUR AURIKULÄREN STIMULATION**
PERSONALIZED AURICULAR STIMULATION ELECTRODE INTERFACE
INTERFACE ÉLECTRODE PERSONNALISÉE DESTINÉE À LA STIMULATION AURICULAIRE

(43) Veröffentlichungstag der Anmeldung: 21.06.2023
(73) Patentinhaber: Hahn-Schickard-Gesellschaft für angewandte Forschung e. V., 70569 Stuttgart (DE); Hochschule Offenburg, 77652 Offenburg (DE)
(72) Erfinder: KELLER, Steffen, 78462 Konstanz (DE); HERRLICH, Simon, 78052 Villingen-Schwenningen (DE); SIKORA, Axel, 79423 Heitersheim (DE)
(74) Vertreter: Hertin und Partner Rechts- und Patentanwälte PartG mbB

(56) Entgegenhaltungen:
- EP-A1- 2 829 259
- WO-A1-2019/005774
- WO-A1-2021/011165
- US-B1- 9 415 220
- US-B2- 10 926 089

## Beschreibung

Die Erfindung betrifft in einem ersten Aspekt eine Vorrichtung zur transkutanen Aufbringung eines elektrischen Stimulationsreizes auf ein Ohr. Die Vorrichtung umfasst einen Schaltungsträger, mindestens zwei Elektroden sowie eine Steuerungseinheit, wobei die Steuerungseinheit dazu konfiguriert ist, anhand von Stimulationsparametern ein elektrisches Stimulationssignal an den Elektroden zu erzeugen. Dabei ist die Vorrichtung, insbesondere eine Oberfläche des Schaltungsträgers der Vorrichtung, auf eine anatomische Form eines Ohres angepasst, sodass Elektroden auf der Oberfläche des Schaltungsträgers aufgebracht sind und ausgewählte Bereiche des Ohres kontaktieren. Insbesondere kann der Schaltungsträger so flexibel sein, dass er sich der Form des Ohres anpassen kann. Die Vorrichtung ist dadurch gekennzeichnet, dass diese weiterhin mindestens einen Sensor zur Erkennung mindestens eines physiologischen Parameters umfasst und eine Steuerungseinheit dazu konfiguriert ist, anhand des mindestens einen physiologischen Parameters die Stimulationsparameter für den Stimulationsreiz anzupassen.

In einem weiteren Aspekt betrifft die Erfindung ein Verfahren zur Herstellung der erfindungsgemäßen Vorrichtung.

### Hintergrund und Stand der Technik

Die transkutane elektrische Nervenstimulation bezeichnet bevorzugt Verfahren, mit denen anhand von auf der Haut angebrachter Elektroden kurze, elektrische Impulse mit niedriger Spannung auf einen oder mehrere Nerven aufgebracht werden. Durch die elektrische Stimulation soll die neuronalen Transmission verändert bzw. aktiviert werden, um beispielsweise das Wohlbefinden zu steigern, Beschwerden zu lindern oder Krankheiten zu behandeln.

Eine besondere Bedeutung kommt der Stimulation von Nervenanteilen des Vagusnervs (VN) im Bereich der Ohren zu. Der Vagusnerv, auch als der 10. Hirnnerv bekannt, ist der längste aller Hirnnerven. Er erstreckt sich vom Hirnstamm durch den Brustbereich, entlang der Luftröhre und bis in den Bauchbereich hinein. Entlang seines Weges zweigt sich der VN zu den einzelnen Organen ab, wie beispielsweise zum Herzen, zu den Lungenflügeln und zum Magen-Darm-Trakt. Der Vagusnerv umfasst viele einzelne Nervenfasern, d. h. Abzweigungen des Vagusnervs, die die einzelnen Organe durchziehen. Insbesondere beinhaltet der Vagusnerv afferente Fasern und efferente Fasern. Afferente Nervenfasern beziehen sich auf sensorische Fasern, d. h. Nervenanteile, die zu erkennende Informationen von einem Sinnesorgan zum Gehirn und/oder Rückenmark übertragen. Efferente Nervenfasern bezeichnen Nervenanteile, die dafür verantwortlich sind, dass motorische Aktionen an die Muskeln übertragen werden, sodass eine Reaktion auf ein bestimmtes Ereignis erfolgen kann.

Der Vagusnerv ist an der Regulation von nahezu allen inneren Organen beteiligt. So steuert er die Aktivität von Herz, Magen-Darm-Trakt, Lunge, Nieren, Leber, Gallenblase, Bauchspeicheldrüse und der Geschlechtsorgane. Umgekehrt leitet er auch Informationen der inneren Organe an das Gehirn weiter. Der Vagusnerv hat zudem Einfluss auf unsere kognitive Leistung, unser Verhalten und unsere Emotionen. Dies kann auf eine Ausschüttung von Botenstoffen im Gehirn, wie z. B. Dopamin, Gamma-Amino-Buttersäure, Serotonin, Adrenalin, Oxytocin und/oder Acetylcholin, basieren.

Weiterhin ist der Vagusnerv ein wichtiger Bestandteil der Darm-Hirn-Achse, also der Verbindung zwischen dem Darm und dem Gehirn.

Durch die Verknüpfung des Vagusnervs mit den inneren Organen und insbesondere dessen Beziehung zum Darm wurde erkannt, dass eine Stimulation des Vagusnervs bei der Behandlung verschiedenster Krankheiten, z. B. Epilepsie, Depressionen, Parkinson, Herzkrankheiten etc. Wirkung zeigen kann. Eine Stimulation des Vagusnervs bei der Behandlung von Krankheiten wird unter dem Ausdruck Vagusnervstimulation (VNS) zusammengefasst. Die VNS bezeichnet bevorzugt eine neuromodulatorische Therapie, bei der der Vagusnerv elektrisch stimuliert wird, d. h., dass insbesondere elektrische Impulse an den Vagusnerv übertragen werden. Eine Behandlung durch eine elektrische Stimulation im Gegensatz zu konventionellen Therapien mittels medikamentöser Behandlungen reduziert das Auftreten unerwünschter Nebenwirkungen oder Kreuzreaktionen.

Im Stand der Technik sind verschiedene Methoden bekannt, die VNS durchzuführen. Typischerweise werden sie eingeteilt in invasive Vagusnervstimulation (iVNS) und nicht-invasive Vagusnervstimulation. Eine nicht-invasive Vagusnervstimulation betrifft insbesondere die transkutane Vagusnervstimulation (tVNS). Das Prinzip der iVNS wird beispielsweise in Ekmek 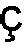 i und Hülagu (2017) beschrieben. Die offenbarte Vorrichtung, welche eine iVNS durchführen kann, weist insbesondere einen Pulsgenerator und einen oder mehrere um den Vagusnerv liegende Elektroden auf. Der Pulsgenerator hat im Wesentlichen die Größe eines Herzschrittmachers und wird unter örtlicher Betäubung oder Narkose im Rahmen einer Operation im Brustbereich des Probanden implantiert. Mithilfe eines (Hand-)Computers kann die Vorrichtung nach der Operation eingeschaltet werden. Des Weiteren kann der Proband einen Magneten erhalten, um oder mit der Unterstützung von anderen Personen die Vorrichtung zu aktivieren, beispielsweise im Falle eines epileptischen Anfalls.

Um chirurgische und implantationsbedingte Risiken zu vermeiden, die mit Vorrichtungen zur iVNS einhergehen, wurden auch Vorrichtungen und Verfahren zur nicht-invasiven Vagusnervstimulation, insbesondere zur transkutanen Vagusnervstimulation (tVNS), entwickelt.

Bei der tVNS werden elektrische Signale durch ein externes Gerät durch die Haut hindurch an den Vagusnerv gesendet. Typischerweise werden diese Geräte am Ohr eines Trägers befestigt, da sich das menschliche Ohr als besonders zugänglich für eine transkutane Stimulation des Vagusnervs gezeigt hat. Im Stand der Technik sind verschiedene Vorrichtungen bekannt, mit denen man eine tVNS durchführen kann.

DE 102005003735 B4 offenbart eine Vorrichtung zur transkutanen Stimulation des Vagusnervs, welche eine Stimulationselektrodeneinheit und eine Steuereinheit zur Festlegung von Stimulationsparametern umfasst. Die Vorrichtung beinhaltet einen bügelförmigen Fortsatz, der über den Oberrand der Ohrmuschel gelegt wird. Weiterhin kann sich die Steuereinheit hinter dem Ohr befinden und die darin befindlichen Elektroden mit Kabeln verbunden werden. Die Verbindung erfolgt ebenfalls über den bügelförmigen Fortsatz. Des Weiteren können Sensoren in der Vorrichtung verbaut sein, die einen Puls oder eine Sauerstoffsättigung messen können. Die gemessenen Werte können auf einem Speicherchip aufgezeichnet und später von einem Arzt ausgelesen und ausgewertet werden. Über die Auswertung kann der Arzt wichtige Informationen über die Wirkung der Vorrichtung erhalten.

Die DE 102006023824 B4 offenbart ebenfalls eine Vorrichtung zur transkutanen Aufbringung eines Stimulationsreizes. Die Vorrichtung ist vollständig in die Ohrmuschel einbringbar, insbesondere die darin befindliche Steuerungselektronik. Dabei ist die Steuerungselektronik in einem Gehäuse untergebracht. Vom Gehäuse aus erstrecken sich zwei gebogene, drahtförmige Abschnitte, die als federelastische Halterungen ausgebildet sind. Die Vorrichtung hat eine C-förmige Gestalt und die beiden gebogenen drahtförmigen Abschnitte weisen an ihren Enden kugelförmige Elemente auf, die als Elektroden fungieren. Weiterhin umfasst die Vorrichtung mindestens einen Sensor oder eine Sensoreinheit, die physiologische oder physikalische Parameter erfasst.

Kaniusas et al. (2019) gibt eine Übersicht über derzeitige Entwicklungen im Forschungsgebiet der Vagusnervstimulation. Unter Anderem wird eine Ausführungsform offenbart, in der Elektroden in Form von Nadeln auf unterschiedliche Bereiche des Ohres platziert werden. Diese Elektroden können in Abhängigkeit eines Messwertes eines Sensors ein angepasstes Stimulationssignal emittieren. Dies erfolgt insbesondere durch die Regulierung der Stärke eines Stimulationssignals der jeweiligen Elektroden.

Allerdings weisen die derzeitigen Vorrichtungen, die einen transkutanen Stimulationsreiz ausgehend vom Ohr eines Menschen an den Vagusnerv emittieren, auch einige Nachteile auf.

EP 2829259 A1 offenbart eine Vorrichtung zur vestibulären Stimulation. Die Vorrichtung umfasst eine Muffe (sleeve), welche in den Ohrkanal eines Patienten einführbar ist. Diese weist eine vorgeformte Form auf. An der Muffe können thermoelektrische Wandler vorliegen, die eine Heizleistung erzeugen. Weiterhin wird auch eine Elektrode offenbart, die ein elektrisches Signal erzeugen kann, d. h. einen elektrischen Stimulationsreiz. Ferner werden Sensoren (u.a. zur Messung des galvanischen Hautwiderstandes) offenbart, auf deren Basis eine Anpassung der Stimulation erfolgen kann.

WO 2021/011165 A1 beschreibt ebenfalls eine Stimulationsvorrichtung, die am Ohr eines Patienten angebracht werden kann. Dazu liegt eine erste Elektrode sowie eine Mehrzahl an Elektroden einer Komponente vor, wobei die Komponente auf bzw. hinter das Ohr angebracht wird. Ferner wird eine automatisierte Anpassung der Therapie beschrieben, die auf ein Feedback der Stimulationsvorrichtung und/oder peripherer Geräte (u.a. *health monitors*) basiert. Insbesondere kann das Feedback auch auf Sensoren zur Überwachung physiologischer Funktionen (u. a. Herzrate, Blutdruck etc.) basieren. Die Anpassung der Stimulation erfolgt durch eine Anpassung der Stimulationsparameter, wie z. B. der Frequenz.

US 10926089 B2 offenbart eine Vorrichtung zur Nervenstimulation am Ohr mit Hilfe einer Kombination von elektrischen, mechanischen und akustischen Stimulationsreizen. Die Intensität der Stimulation kann anhand eines Feedbacks von Sensoren in Echtzeit erhöht oder verringert werden. Hierbei werden auch Sensoren offenbart, um physiologische Parameter zu messen (insbesondere in Bezug auf kardiovaskuläre Funktionen). Die Erfassung der physiologischen Parametern erfolgt durch eine oder mehrere Messelektroden. Die Vorrichtung umfasst eine Stütze, auf der die Komponenten angebracht sind sowie einen Ohrkanalabschnitt, welcher die Form eines Ohrkanals aufweisen kann und durch Abformung eines Ohres bereitstellbar ist. In einer weiteren Ausführungsform umfasst die Vorrichtung einen Magnet oder Klebstoff, um eine stabile Anbringung zu ermöglichen.

WO 2019/005774 A1 offenbart eine Vorrichtung zur transdermalen Stimulation des peripheren Nervensystems, insbesondere eines Zweiges des Vagusnerves am Ohr. Die Stimulation erfolgt durch einen elektrischen Strom, welcher von mindestens zwei Elektroden ausgeht, der Stimulationselektrode und der Referenzelektrode. Die Vorrichtung weist ein C-förmiges Interface auf, welcher mit den Elektroden verbunden ist. Die Elektroden sollen eine hinreichende Flexibilität aufweisen, um eine Anpassung an der Anatomie von Abschnitten des Ohres zu ermöglichen. In allgemeiner Form wird zudem dargelegt, dass die Vorrichtung Merkmale aufweisen solle, die eine optimale Anpassung am Ohr unter Berücksichtigung der anatomischen Geometrie ermöglichen. Die Vorrichtung kann einen Sensor aufweisen, um einen physiologischen Parameter des Patienten zu messen (u. a. Blutdruck, Sauerstoffsättigung, Herzrate). Eine Kontrolleinheit der Vorrichtung kann weiterhin dazu eingerichtet sein, in Abhängigkeit der durch den Sensor aufgenommenen Messdaten Stimulationsparameter im Hinblick auf eine Verbesserung der Therapie anzupassen.

US 9415220 B1 betrifft eine Vorrichtung zur aurikulären Stimulation eines Patienten. Hierbei wird die Verwendung von Sensoren für ein Feedback offenbart, wobei sich das Feedback auf eine korrekte Platzierung der Elektroden z.B. anhand einer Impedanzmessung bezieht. Zur Formgebung der Vorrichtung kann beispielsweise die Morphologie eines Ohrläppchens gemessen werden. Hierbei kann auch ein 3D-Modell des Ohres gebildet werden. Des Weiteren wird offenbart, dass die Form der Stimulationsvorrichtung oder Positionierung der Elektroden unter Berücksichtigung vorheriger Therapieantworten ausgewählt bzw. modelliert werden kann.

Durch die bekannten Platzierungen der Vorrichtung mittels Bügelfortsätzen oder Einbringungen in der Ohrmuschel liegt nicht immer ein nötiger Halt vor, welche eine Nutzung in alltäglichen Situationen oder beim Sport, erlaubt. Die Bereitstellung von Elektroden, die als Nadeln in den Ohren befestigt werden gemäß Kaniusas et al. (2019) beugen einem Verrutschen der Elektroden vor, wirken sich jedoch nachteilig auf den Tragekomfort aus und erlauben keinen Einsatz außerhalb eines medizinisch betreuten Settings.

Weiterhin ist es von Nachteil, dass das Stimulationssignal nur in eingeschränktem Maße auf individuelle Bedürfnisse eines Patienten angepasst werden kann. Oftmals ist eine Konsultation bei einem Arzt oder einem anderen medizinischen Personal notwendig, welches die aufgenommenen Daten auswerten muss, um ein therapeutisch effektives Stimulationsmuster einzustellen bzw. nachzujustieren. Dies ist aufwändig und kann zu einem Zeitverlust führen, welche den Behandlungserfolg vermindert.

Mithin besteht ein Bedarf an alternativen Vorrichtungen zur Vagusnervstimulation, welche die Stimulation an individuelle Bedürfnisse der Probanden optimal anpassen kann und sich gleichzeitig durch einen stabilen, dauerhaften und robusten Tragekomfort auszeichnen.

### Aufgabe der Erfindung

Aufgabe der Erfindung war es, die Nachteile des Standes der Technik zu beseitigen und eine verbesserte Vorrichtung zur transkutanen Aufbringung eines Stimulationsreizes bereitzustellen. Insbesondere sollte die Vorrichtung in der Lage sein, ein auf individuelle Bedürfnisse eines Trägers optimiertes Stimulationssignal zu erzeugen und eine gezielte Stimulation der Nervenfasern des Vagusnervs zu gewährleisten. Weiterhin sollte sich die Vorrichtung durch einen stabilen, dauerhaften und robusten Tragekomfort auszeichnen, sodass die Vagusnervstimulation besonders einfach in den Alltag integriert werden kann und eine hohe Compliance (Bereitschaft zur Mitwirkung) sowie ein effizienter Behandlungserfolg erreicht wird.

### Zusammenfassung der Erfindung

Die erfindungsgemäße Aufgabe wird gelöst durch die Merkmale der unabhängigen Ansprüche. Vorteilhafte Ausgestaltungen der Erfindung sind in den abhängigen Ansprüchen beschrieben.

In einem ersten Aspekt betrifft die Erfindung eine Vorrichtung zur transkutanen Aufbringung eines elektrischen Stimulationsreizes auf ein Ohr umfassend einen Schaltungsträger, mindestens zwei Elektroden sowie eine Steuerungseinheit, wobei eine Oberfläche des Schaltungsträgers an eine anatomische Form des Ohres angepasst ist und die Elektroden derart auf der Oberfläche des Schaltungsträgers aufgebracht vorliegen, dass die Elektroden mit Bereichen des Ohres kontaktiert sind, wobei die Steuerungseinheit dazu konfiguriert ist, anhand von Stimulationsparametern ein elektrisches Stimulationssignal an den Elektroden zu erzeugen, wobei die Vorrichtung mindestens einen ersten Sensor zur Erkennung mindestens eines physiologischen Parameters umfasst und die Steuerungseinheit dazu konfiguriert ist, anhand einer Messung des physiologischen Parameters die Stimulationsparameter anzupassen, wobei die Vorrichtung mindestens drei oder mehr Elektroden aufweist, dadurch gekennzeichnet, dass die drei oder mehr Elektroden als Array angeordnet vorliegen, wobei mittels der Steuerungseinheit die Elektroden einzeln steuerbar sind und die Steuerungseinheit dazu konfiguriert ist, anhand einer Auswertung des gemessenen physiologischen Parameters eine Auswahl von Elektroden zu ermitteln, welche besonders geeignet sind, durch einen elektrischen Stimulationsreiz eine Veränderung des physiologischen Parameters zu bewirken.

Die erfindungsgemäße Vorrichtung zeichnet sich dadurch aus, dass der Schaltungsträger mit hoher Präzision, insbesondere passgenau, am Ohr eines Trägers angebracht werden kann. Durch eine Anpassung an eine anatomische Form des Ohres kann die Vorrichtung vorzugsweise zudem individuell an beliebige Formen und/oder Gestaltungen von Ohren platziert werden.

Vorteilhafterweise kann durch die anatomische Anpassung des Schaltungsträgers auf das Ohr zudem eine enganliegende und stabile an oder im Ohr sitzende Vorrichtung bereitgestellt werden, die von Trägern in alltäglichen Situationen getragen werden kann. Beispielsweise kann die Vorrichtung sowohl während Arbeits- als auch Freizeittätigkeiten, sogar beim Betreiben von Sport, getragen werden, während durch die präzise Positionierung des Schaltungsträgers und mithin der Elektroden eine effektive VNS gesichert ist. Die Vorrichtung kann somit insbesondere über längere Zeiträume getragen werden, sodass für einen langanhaltenden Therapiezeitraum eine zuverlässige Aufbringung des Stimulationssignales gewährleistet wird.

Weiterhin kann vorteilhaft die erfindungsgemäße Vorrichtung vom Träger selbst an und/oder in das Ohr positioniert werden. Eine Anbringung durch medizinisches Personal - wie im Falle von der Platzierung von stimulierenden Nadeln - ist nicht notwendig. Somit wird durch die erfindungsgemäße Vorrichtung eine besonders einfache, mühelose und sichere Platzierung ermöglicht.

Weiterhin zeichnet sich die Vorrichtung dadurch aus, dass anhand der Messung physiologischer Parameter ein auf den Träger optimales und angepasstes Stimulationsmuster für den Stimulationsreiz bereitgestellt werden kann. Die Messung der physiologischen Parameter erlaubt insbesondere eine Reaktion des Körpers auf die VNS zu erkennen und die Stimulation daraufhin automatisiert anzupassen. Insbesondere wird zudem eine Regelung der Stimulationsparameter anhand eines geschlossenen Regelkreis ermöglicht, welcher gezielt auf eine Beeinflussung einer oder mehrerer physiologischer Parameter zielt. Die Anpassung der Stimulationsparameter anhand aufgenommenen physiologischen Parametern erfolgt vorteilhaft automatisiert, sodass keine zusätzliche Auswertung aufgenommener durch einen Arzt oder ein medizinisches Personal notwendig ist. Dies spart nicht nur Zeit und Kosten, sondern erlaubt eine kontinuierliche Optimierung der VNS auf ein Behandlungsziel über langanhaltende Zeiträume, wodurch sich der Behandlungserfolg erhöht.

Die erfindungsgemäße Vorrichtung kann überaus effizient auf nicht-invasive Weise durch Stimulation von Nervenfasern und/oder Nervenbündel, insbesondere des Vagusnervs, für ein verbessertes Wohlbefinden sorgen oder gezielt Krankheiten behandeln. Dabei kann die Aufbringung des Stimulationsreizes bevorzugt anhand aufgenommener physiologischer Parameter kontinuierlich angepasst werden.

Physiologische Parameter können hierbei mit einem Stadium, einer Intensität und/oder Prognose eines Krankheitsverlaufs korrelieren. So ist es beispielsweise bekannt, dass von Normwerten abweichende Herzfrequenzen (z. B. erhöhte Herzfrequenzen) Symptome für evtl. auftretende Schlaganfälle und/oder Herzinfarkte sind. Ebenso ist es in Bezug auf weitere medizinische Beschwerden wie beispielsweise Bluthochdruck, Demenz, Migräne, Parkinson und/oder ein Entzug bekannt, dass diese mit von Normwerten abweichenden physiologischen Parametern zusammenhängen. Weitere Beispiele können auch chronische Schmerzen (z. B. Kopfschmerzen) Atemwegserkrankungen, Verdauungsbeschwerden, wie beispielsweise das Reizdarmsyndrom und/oder Störungen der Magenbeweglichkeit sein. Des Weiteren können von Normwerten abweichende physiologische Parameter auch psychische Erkrankungen betreffen, wie beispielsweise Angstzustände und/oder Depressionen.

Vorteilhaft kann die erfindungsgemäße Vorrichtung durch Aufbringung von elektrischen Stimulationsreizen eine gezielte Veränderung physiologischen Parameter herbeiführen und hierdurch einen positiven Einfluss auf einen Krankheitsverlauf nehmen. Vorteilhaft muss die Anpassung der Stimulationsparameter in Abhängigkeit der gemessenen physiologischen Parameter nicht extern vorgegeben werden. Stattdessen kann durch eine Regelkreissteuerung die Vorrichtung selbstständig durch Variation von Stimulationsparameter optimierte Behandlungsbereiche festzulegen. Zu diesem Zweck können auch Algorithmen der künstlichen Intelligenz Einsatz finden, um Stimulationsparameter zu ermitteln, welche besonders effektiv eine gewünschte Veränderung eines physiologischen Parameters bewirken.

Die anatomische Anpassung des Schaltungsträgers, an dessen Oberfläche sich die Elektroden befinden, ist zudem dahingehend von Vorteil, dass die Elektroden besonders gezielt und präzise Stimulationsreize auf Nervenfasern, insbesondere des Vagusnervs, übermitteln können. Hierbei kann es bevorzugt sein, dass die Vorrichtung durch die Anpassung auf die anatomische Form des Ohres auch insbesondere einen individuellen Verlauf von Nervenfasern berücksichtigt, um eine optimale Übertragung des Stimulationsreizes auf die Nervenfasern zu gewährleisten.

Ein Schaltungsträger ist bevorzugt ein Bauelement umfassend ein elektrisch isolierendes Material (Substrat), auf dem elektrisch leitende Verbindungen (Leiterbahnen) und/oder elektronische Bauelemente bzw. Baugruppen vorliegen. Ein Schaltungsträger bezeichnet mithin vorzugsweise einen Träger für elektronische Bauteile oder elektrisch leitende Verbindungen, der sowohl der mechanischen Befestigung als auch der elektrischen Verbindung dient. Da auf oder in dem Schaltungsträger Komponenten wie die Steuerungseinheit, die Elektroden und der Sensor aufgebracht sind, liegen insbesondere elektrische Verbindungen vor, die beispielsweise durch Drahtbonds und/oder Leiterbahnen vermittelt werden. Somit fungiert ein Substrat des Schaltungsträges als mechanischer Träger und kann zeitgleich elektrische Funktionen realisieren, wie beispielsweise elektrische Verbindungen für einzelne Komponenten bereitzustellen. Der Schaltungsträger kann elektronische Schaltungen aufweisen, um die Funktionstüchtigkeit der Komponenten zu ermöglichen. Vorzugsweise wird der Schaltungsträger am Ohr eines Nutzers angebracht, um einen transkutanen Stimulationsreiz aufzubringen, insbesondere um den Vagusnerv zu stimulieren.

In bevorzugten Ausführungsformen ist der Schaltungsträger eine dreidimensionale flexible Leiterplatte, welche, wie hierin erläutert, durch eine digitale Prozesskette unter Verwendung beispielsweise additive Fertigungsverfahren, bevorzugt einen hybriden Aufbau aufweist. Insbesondere können Fertigungsverfahren, welche unter dem Begriff des Urformens zusammengefasst werden können zum Einsatz kommen, bei denen aus einem formlosen Stoff ein fester Körper hergestellt wird, der eine geometrisch definierte Form hat. Besonders bevorzugt ist ein additives Fertigungsverfahren. Letzteres hat sich als besonders prozesseffizient erwiesen, um hinsichtlich geometrischen Ausgestaltung und/oder Materialauswahl den Schaltungsträger in einer digitalen Prozesskette bereitzustellen.

Dabei ist es bevorzugt, dass der Schaltungsträger mehrere Materialien (Multimaterial) aufweist. So werden vorzugsweise elektrisch leitende und elektrisch isolierende Materialien eingesetzt, die der Schaltungsträger aufweisen kann. Die elektrisch leitenden Materialien werden bevorzugt verwandt, um beispielsweise Leiterbahnen bereitstellen zu können für den Kontakt zwischen Steuerungseinheit, Elektroden und/oder dem mindestens einen ersten Sensor oder die Elektroden selbst. Die elektrisch isolierenden Materialien bilden hingegen bevorzugt die Grundform des Schaltungsträgers, um einen mechanischen Kontakt mit Abschnitten des Ohres zu gewährleisten, während an den Elektroden ein elektrischer Stimulationsreiz aufgebracht wird. In bevorzugten Ausführungsformen können die nicht elektrisch leitenden Materialien elastisch verformbar ausgestaltet werden, um eine besonders robuste Positionierung der Vorrichtung in oder auf dem Ohr zu unterstützen.

Der Schaltungsträger kann auch in Form eines Gehäuses vorliegen, sodass die Komponenten innerhalb und/oder außerhalb des Gehäuses vorliegen. Bevorzugt werden die Elektroden derart auf oder in den Schaltungsträger angebracht, dass ein direkter Kontakt mit der Hautoberfläche des Ohres bereitgestellt wird. Bevorzugt umfasst der Schaltungsträger biokompatibles Material, sodass vorteilhaft eine Reizung der Haut vermieden wird. Auch der mindestens eine erste Sensor zur Messung eines physiologischen Parameters kann derart platziert werden, dass er mit der Hautoberfläche direkt kontaktiert werden kann.

In bevorzugten Ausführungsformen ist der Schaltungsträger flexibel ausgestaltet, sodass der Schaltungsträger vorzugsweise durch Ausübung einer Kraft noch präziser an eine anatomische Form des Ohres angepasst werden kann. Zu diesem Zweck umfasst der Schaltungsträger vorzugsweise ein flexibles Trägermaterial, auf dessen Oberfläche die Elektroden und ggf. Leiterbahnen angeordnet sind. Eine Flexibilität des Schaltungsträgers meint insbesondere eine Verformbarkeit, d. h. einen hinreichend geringen mechanischen Widerstand des Materials gegenüber Formveränderung, beispielsweise durch Ausübung eines Druckes oder Zuges durch einen Nutzer beim Einbringen des Schaltungsträgers in oder an das Ohr. Die Verformung kann vorzugsweise elastisch und/oder plastisch erfolgen.

In bevorzugten Ausführungsformen ist der Schaltungsträger streck- und/oder stauchbar. Vorteilhaft durch ein Strecken und/oder Stauchen des Schaltungsträgers eine geeignetere Oberfläche bereitgestellt werden, um eine optimierte Passgenauigkeit zu gewährleisten. Zudem gewährleistet eine Streck- und/oder Stauchbarkeit des Schaltungsträgers, dass auch bei geringfügigen Verformungen des Ohres stets eine stabile enganliegende Kontaktierung am Ohr erreicht wird.

Auf der Oberfläche des Schaltungsträges befinden sich mindestens drei oder mehr Elektroden, wobei diese dazu dienen, einen elektrischen Strom bereitzustellen, um die Stimulation von Nervenfasern vorzunehmen. Vorzugsweise handelt es sich um mindestens eine Anode und Kathode, um einen geschlossenen Stromkreis zu erhalten. Die Kathode ist die Gegenelektrode zur Anode, d. h. insbesondere, dass Kationen zur Kathode wandern und Anionen zur Anode. Dabei ist dem durchschnittlichen Fachmann klar, dass durch die Verwendung der Formulierung, dass ein elektrischer Strom emittiert wird oder ein elektrisches Stimulationssignal angelegt wird, bevorzugt ein Stromfluss in Form eines geschlossenen Stromkreises gemeint ist. Vorzugsweise wird der elektrische Stromfluss bzw. das elektrische Stimulationssignal durch die Elektroden ermöglicht.

Vorzugsweise bezeichnet ein elektrischer Stimulationsreiz ein elektrisches Signal, dass insbesondere durch die Steuerungseinheit herbeigeführt und ausgehend von den Elektroden emittiert wird, um insbesondere Nervenanteile und/oder Nervenbündel des Vagusnervs zu stimulieren. Dabei können die Formulierungen Stimulationsreiz und Stimulationsreiz im erfindungsgemäßen Kontext synonym verwendet werden.

Dass die Oberfläche des Schaltungsträgers an eine anatomische Form des Ohres angepasst ist, meint bevorzugt, dass insbesondere die Form des Schaltungsträgers bereichsweise an mindestens einen Abschnitt eines Ohres angepasst bereitgestellt wird. Der Begriff eine anatomische Form meint mithin bevorzugt eine Form eines Teilbereiches oder Abschnittes des Ohres. Das Ohr umfasst verschiedene Abschnitte zur Ausführung seiner Funktion. Der prägnanteste Abschnitt ist das Außenohr, der die Ohrmuschel und den äußeren Gehörgang umfasst. Die Ohrmuschel fängt Schallwellen auf und leitet sie durch den äußeren Gehörgang zum Trommelfell weiter. Die Ohrmuschel stellt den größten sichtbaren Abschnitt des Ohres dar, welcher wiederrum in eigene Bereiche eingeteilt werden kann. Eine Anpassung der Oberfläche des Schaltungsträgers kann beispielsweise mindestens bereichsweise an die Ohrmuschel erfolgen, sodass die Elektroden Nervenenden des Vagusnervs im Bereich der Ohrmuschel stimulieren können. Ebenso ist eine Anpassung der Oberfläche des Schaltungsträgers in Bezug auf den äußeren Gehörgang möglich, sodass die Vorrichtung gleich einem In-Ear-Kopfhörer getragen werden kann.

Bevorzugt handelt es sich bei dem Ohr um ein menschliches Ohr. Die Anatomie des menschlichen Ohres fällt von Mensch zu Mensch unterschiedlich aus, weist jedoch auch Gemeinsamkeiten auf, welche eine gewisse Standardisierung der Anpassung der anatomischen Form erlauben.

Die Anpassung der Oberfläche des Schaltungsträgers an eine anatomische Form des Ohres, vorzugsweise menschlichen Ohres, kann sich mithin auf eine Anpassung auf einen Model eines menschlichen Ohres beziehen, welche durch Mittelung der charakteristischen Form einer Vielzahl verschiedener menschlicher Ohren erhalten wurde. Hierbei können selbstverständlich auch Untergruppierungen für Modelle menschlicher Ohren bereitgestellt werden, welche beispielsweise eines Alters oder geschlechtsspezifische unterschiedliche Dimensionierungen beinhalten. Besonders bevorzugt erfolgt die Anpassung der Oberfläche des Schaltungsträgers mindestens abschnittsweise an eine individuelle anatomische Form eines menschlichen Ohres, wodurch eine besondere hohe Passgenauigkeit und Effizienz der Nervenstimulation gewährleistet werden kann.

In bevorzugten Ausführungsformen ist die Anpassung des Schaltungsträgers an eine anatomische Form des Ohres im Wesentlichen durch eine Erstreckung des Schaltungsträgers auf oder an der Ohrmuschel gegeben, wobei der Schaltungsträger vorzugsweise auf Abschnitte der Ohrmuschel anatomisch angepasst ist, beispielsweise an die Concha. Insbesondere kann der Schaltungsträger derart ausgestaltet sein, dass er sich entlang mehrerer Abschnitte der Ohrmuschel erstreckt. Somit kann die Anpassung des Schaltungsträger auf die Anatomie des Ohres im Wesentlichen die gesamte Ohrmuschel oder Teilbereiche der Ohrmuschel betreffen. Ebenso kann eine Anpassung an einen Bereich des äußeren Gehörgangs bevorzugt sein.

Die Anpassung des Schaltungsträgers auf eine anatomische Form des Ohres bezeichnet somit insbesondere eine im Wesentlichen in Form und/oder Größe eines Bereiches des Ohres, insbesondere kongruente, Abdeckung. Vorteilhafterweise wird eine besonders stabile und passgenaue Vorrichtung bereitgestellt, die intraindividuell an die Form von Ohren der Nutzer angepasst ist.

In bevorzugten Ausführungsformen erfolgt eine Anpassung auf eine anatomische Form des Ohres erfolgt durch ein Abbildungsverfahren des Ohres. Beispielsweise wird dazu in einem Abbildungsverfahren ein dreidimensionales Bild eines Teilbereiches des Ohr aufgenommen und der Schaltungsträger auf Basis des dreidimensionalen Bildes hergestellt. Falls es bevorzugt ist, dass der Schaltungsträger sich auf bestimmte Abschnitte oder Teilbereiche der Ohrmuschel angepasst wird, wird vorzugsweise ein dreidimensionales Bild von den entsprechenden Abschnitten oder Teilbereichen des Ohrmuschel erstellt, auf deren Grundlage der Schaltungsträger dann bereitgestellt wird. Ebenso kann eine Anpassung an einen äußeren Gehörgang erfolgen, wobei ein Abbildungsverfahren der Form des äußeren Gehörgangs vorzugsweise zum Einsatz kommt. Vorteilhaft kann somit der Schaltungsträger besonders präzise an die Anatomie eines menschlichen Ohres angepasst werden. Hierbei kann sowohl ein Modell erstellt werden, welches eine Mittelung einer Mehrzahl verschiedener menschliche Ohren (bzw. deren Teilbereich) darstellt oder aber es wird die individuelle Anatomie des Trägers berücksichtigt. Folglich wird ein optimaler und dauerhafter Halt gesichert und weiterhin ein angenehmer Tragekomfort für den Nutzer geschaffen.

Der Schaltungsträger hat mithin in einigen Ausführungsformen hinsichtlich seines äußeren Erscheinungsbildes die Form eines Hörgerätes. In weiteren Ausführungsformen hat der Schaltungsträger die Gestaltung eines In-Ear-Kopfhörers. Die Anpassung auf eine anatomische Form des Ohres schließt beide Gestaltungsvarianten ein.

In besonders bevorzugten Ausführungsformen umfasst eine Anpassung der Oberfläche des Schaltungsträgers auf eine anatomische Form des Ohres insbesondere auch eine Positionierung der Elektroden, welche den Verlauf der Nervenfasern des Vagusnervs im Ohr berücksichtigt. Vorzugsweise kann diese anhand einer vorherigen Bestimmung der Positionierung der Nervenfasern des Vagusnervs unterhalb der Hautoberfläche erfolgen. Vorteilhafterweise wird hierdurch der Stimulationsreiz besonders präzise an die Nervenfasern übertragen, sodass eine besonders optimierte und zuverlässige Behandlung garantiert werden kann.

Im Sinne der Erfindung bezeichnet eine Steuerungseinheit eine Einheit, die dazu eingerichtet ist, den von den Elektroden ausgehenden Stimulationsreiz vorzugeben bzw. zu erzeugen. Die Steuereinheit umfasst zu diesem Zweck bevorzugt eine elektronische Schaltung, welche bevorzugt dazu eingerichtet ist, anhand von Stimulationsparametern ein elektrisches Stimulationssignal an den Elektroden erzeugen.

Bevorzugte elektronische Schaltungen umfassen ohne Beschränkung eine integrierte Schaltung (IC), eine anwendungsspezifische integrierte Schaltung (ASIC), eine programmierbare logische Schaltung (PLD), ein Field Programmable Gate Array (FPGA), einen Mikroprozessor, einen Mikrocomputer, eine speicherprogrammierbare Steuerung und/oder eine sonstige elektronische, bevorzugt programmierbare, Schaltung.

Die Steuerungseinheit ist somit bevorzugt zudem eine Prozessoreinheit und mithin zur Datenverarbeitung geeignet. Die Steuerungseinheit ist bevorzugt geeignet zum Einlesen, zur Verarbeitung und/oder zur Ausgabe elektronischer Signale, welche durch die Konfiguration der Steuerungseinheit bestimmt werden. Die Speichereinheit erlaubt die Sicherung und/oder Zwischenspeicherung von Daten. Nicht beschränkende Beispiele von Speichern, vorzugsweise Halbleiterspeicher, sind flüchtige Speicher, (RAM)-Speicher oder nicht-flüchtige Speicher, wie ROM-Speicher, EPROM-Speicher, EEPROM-Speicher oder Flash-Speicher und/oder andere Speichertechnologien. So kann es bevorzugt sein, dass physiologische Parameter, Stimulationsparameter und/oder ein Stimulationsmuster auf der Speichereinheit hinterlegt werden.

Der Ausdruck, die Steuerungseinheit ist konfiguriert (bzw. eingerichtet oder ähnliche Formulierungen) zur Ausführung bestimmter Verfahrensschritte oder Berechnungen meint bevorzugt, dass auf der Steuerungseinheit (bzw. deren Speicher), eine Software, Firmware bzw. ein Computerprogramm gespeichert vorliegt, welches Befehle umfasst, die Verfahrens- bzw. Berechnungsschritte ausführen.

Die Steuerungseinheit ist dazu eingerichtet, anhand einer Messung von einem oder mehreren physiologischen Parametern die Stimulationsparameter zur Erzeugung eines elektrischen Stimulationssignals an den Elektroden anzupassen.

Stimulationsparameter sind bevorzugt Parameter, die den elektrischen Stimulationsreiz beschreiben und/oder charakterisieren können. Ein Stimulationsparameter umfasst daher beispielsweise eine Stromstärke, eine zeitliche Dauer eines elektrischen Impulses, Stimulationsintervalle, eine Amplitude und/oder eine Frequenz.

Der mindestens eine erste Sensor bezeichnet bevorzugt einen Sensor, der mindestens einen, bevorzugt mehrere, physiologische Parameter misst. Physiologische Parameter bezeichnen vorzugsweise Größen, die die Funktionen des menschlichen Körpers widerspiegeln und/oder anhand derer Rückschlüsse über den Gesundheitszustand ermöglicht werden. Physiologische Parameter umfassen beispielsweise einen Blutdruck, eine Sauerstoffsättigung, eine Körpertemperatur, eine Herzfrequenz und/oder einen pH-Wert. Im Falle einer Beeinträchtigung eines Gesundheitszustands ist es bekannt, dass ein oder mehrere physiologische Parameter gegenüber einem Normalzustand verändert sind. Eine Veränderung physiologischer Parameter kann sowohl bei physischen (beispielsweise kardiovaskuläre Erkrankungen, Migräne, Verdauungsbeschwerden, Lebererkrankungen und/oder Milzerkrankungen etc.) als auch psychischen Erkrankungen (beispielsweise Angstzustände, Depressionen, Traumabewältigung, Depressionen, Panikattacken und/oder Schizophrenie etc.) präsent sein. Vorteilhafterweise führt die Anpassung der Stimulationsparameter für die VNS im Hinblick auf eben jene physiologischen Parameter - vorzugsweise zur Führung der physiologischen Parameter in einen Normbereich - zu einer kontinuierlichen Verbesserung des Gesundheitszustandes und Wohlbefindens.

Der mindestens eine erste Sensor ist bevorzugt dazu eingerichtet, innerhalb von mehreren Zeitpunkten, von bestimmten Zeitbereichen und/oder dauerhaft, insbesondere kontinuierlich, eine Messung von einen oder mehreren physiologischen Parametern vorzunehmen. In bevorzugten Ausführungsformen kann der mindestens eine erste Sensor einen oder mehrere physiologische Parameter innerhalb von Sekunden, Minuten oder Stunden aufnehmen. Weiterhin kann es bevorzugt sein, über einen Zeitraum von Tagen, Wochen oder Monaten einen oder mehrere physiologische Parameter zu messen. Beispielsweise kann es bevorzugt sein, für einen Monat alle vier Stunden eine Messung für einen oder mehrere physiologische Parameter vorzunehmen. Die gesamte Messdauer und der Zeitrahmen, in denen ein oder mehrere physiologische Parameter gemessen werden, hängt von den jeweiligen Beschwerden und den Probanden ab.

Dabei bezeichnet ein Träger oder Nutzer vorzugsweise eine Person, welche die erfindungsgemäße Vorrichtung zur Durchführung einer VNS verwendet. In bevorzugten Ausführungsformen kann der Nutzer oder Träger ein Patient sein, der eine physische und/oder psychische Erkrankung aufweist, die mittels der erfindungsgemäßen Vorrichtung behandelt werden soll. In weiteren bevorzugten Ausführungsformen kann der Träger oder Nutzer auch eine gesunde Person sein, welche die erfindungsgemäße Vorrichtung nicht zur Behandlung einer Krankheit, sondern zur Steigerung des individuellen Wohlbefindens verwendet.

In bevorzugten Ausführungsformen weist die Vorrichtung eine elektrische Energiequelle auf, um die Stromversorgung zu gewährleisten. Die Energiequelle kann beispielsweise bereitgestellt werden durch eine Batterie und/oder einen Akkumulator. Bevorzugt befindet sich die Energiequelle an oder innerhalb des Schaltungsträgers.

In einer weiteren bevorzugten Ausführungsform ist die Vorrichtung dadurch gekennzeichnet, dass eine Anpassung der Stimulationsparameter anhand der von gemessenen physiologischen Parameter in einem geschlossenen Regelkreis erfolgt, wobei bevorzugt die Anpassung der Stimulationsparameter zur Führung des physiologischen Parameters auf einen vorgegeben Zielwert führt.

Vorteilhafterweise wird insbesondere durch den geschlossenen Regelkreis ein auf den Gesundheitszustand und/oder Wohlbefinden des Nutzers individualisiertes, optimales und präzise angepasstes Stimulationssignal aufgebracht. Insbesondere beruht die Aufbringung des Stimulationsreizes durch den geschlossenen Regelkreis auf eine Rückmeldung des Körpers auf zuvor aufgebrachte Stimulationsreize.

Vorteilhafterweise wird eine erhöhte Behandlungseffizienz erreicht, da direkt, insbesondere durch die Vorrichtung selbst, eine Anpassung von Stimulationsparametern anhand der von dem ersten Sensor aufgenommenen physiologischen Parametern erfolgt. Vorteilhaft ist es somit nicht mehr notwendig, dass beispielsweise ein Arzt aufgenommene physiologische Parameter auswertet und nach einer Analyse der aufgenommenen physiologischen Parameter ein Stimulationssignal anpasst. Stattdessen nimmt die Vorrichtung selbst eine Auswertung der physiologischen Parameter vor, bevorzugt durch die Steuerungseinheit, woraufhin ein individualisiertes Stimulationssignal am menschlichen Ohr aufgebracht wird. Insbesondere werden zeitaufwendige Arztbesuche durch die erfindungsgemäße Vorrichtung vermieden. Vorteilhaft kann zudem eine kontinuierliche Optimierung der Behandlung gewährleistet werden, welche im Falle eines langfristige Tragens der Vorrichtung besonders vorteilhaft ist. Durch die Bereitstellung eines auf den jeweiligen Nutzer bzw. dessen Bedürfnissen abgestimmten Stimulationssignales wird der Behandlungserfolg erhöht und die Behandlungsdauer verkürzt.

Besonders vorteilhaft ist der Umstand, dass eine dynamische Anpassung des Stimulationssignals durch den geschlossenen Regelkreis gegeben ist. Hierbei bezeichnet eine dynamische Anpassung des Stimulationssignals bevorzugt, dass das Stimulationssignal zu jeder Zeit während der Behandlung angepasst werden kann. Insbesondere kann die Anpassung des Stimulationssignals automatisch erfolgen, da die Steuerungseinheit dazu vorzugsweise eingerichtet ist, einen Stimulationsreiz mit geeigneten Stimulationsparametern zu ermitteln. Die Ermittlung des optimalen Stimulationsreizes wird bevorzugt anhand aufgenommener physiologischer Parameter durchgeführt. Insbesondere wird vorteilhaft berücksichtigt, wie der Träger auf zuvor aufgebrachte Stimulationssignale reagiert. Die Reaktion des Trägers auf die VNS in Form einer Veränderung des physiologischen Parameters kann in einigen Fällen in unmittelbarer zeitlicher Nähe mit der VNS erfolgen, beispielsweise im Wesentlichen simultan oder innerhalb von wenigen Minuten. Ebenso kann die physiologische Reaktion auf die VNS auch in Form einer Veränderung des physiologischen Parameters mit einem zeitlichen Versatz von mehreren Stunden oder mehreren Tagen resultieren. Der geschlossene Regelkreis kann vorteilhaft gleichermaßen auf kurzfristige wie auch langfristige Veränderungen der physiologischen Parameter durch Anpassung der Stimulationsparameter Einfluss nehmen.

Vorteilhaft wird durch die Messung der physiologischen Parameter (und der Anpassung der Stimulationsparameter) eine objektivierte Ausrichtung der Behandlung ermöglicht, wobei die Veränderungen der physiologischen Parameter als frühzeitige Indikatoren für eine therapeutisch effektives (oder weniger effektiven) Stimulationsmuster dienen. In der Regel werden sich physiologische Parameter wie beispielsweise eine Herzfrequenz, Sauerstoffsättigung, eine Körpertemperatur, eine Herzfrequenz oder ein pH-Wert in messbaren Maße zeitlich deutlich früher ändern, als dass ein Träger subjektiv eine Verbesserung empfindet und/oder klinische Symptome einer Krankheit sich sichtbar verändern.

Dem durchschnittlichen Fachmann ist bekannt, dass durch einen geschlossenen Regelkreis im Allgemeinen eine vorgegebene Größe (Regelgröße) auf einen gewünschten Wert (Sollwert) gebracht werden soll, indem ein Istwert gemessen und nachgeregelt wird.

Im erfindungsgemäßen Kontext ist die Regelgröße vorzugsweise mindestens ein physiologischer Parameter, der mit mindestens einer physischen und/oder physischen Erkrankung zusammenhängt und die nachgeregelt werden soll, um die mindestens eine Erkrankung erfolgreich zu behandeln. Der Istwert des physiologischen Parameters wird bevorzugt durch den ersten Sensor gemessen. Innerhalb der Steuerungseinheit liegen vorzugsweise Werte und/oder Wertebereiche von physiologischen Parametern vor, die einen wünschenswerten Zustand beschreiben. Diese Werte und/oder Wertebereiche, auf die der physiologische Parameter gebracht werden soll, entsprechen den vorgegebenen Zielwerten. Ist der Zielwert dauerhaft erreicht, gilt die individuelle Behandlung als erfolgreich eingestellt. Nach dauerhaften erfolgreichem Erreichen eines Zielwertes oder Zielbereiches kann es bevorzugt sein, die Behandlungsdauer zu reduzieren und/oder zu beenden. Dies wird im Einzelfall von dem erreichten subjektiven Wohlbefinden, sowie ggf. in Begutachtung durch medizinisches Personal erfolgen.

Zum Erreichen eines Zielwertes bzw. Sollwertes wird bevorzugt durch die erfindungsgemäße Vorrichtung der entsprechende physiologische Parameter (Regelparameter) sukzessive auf den Zielwert geführt. Ausgehend von den Elektroden wird ein elektrischer Stimulationsreiz aufgebracht mit dem Ziel, einen bestimmten physiologischen Parameter auf einen gewünschten Wert, den Sollwert, zu regeln. Der momentane Wert des physiologischen Parameters, der Istwert, wird durch den ersten Sensor gemessen. Anhand des in der Steuerungseinheit befindlichen Zielwertes ist bevorzugt die Steuerungseinheit dazu eingerichtet, eine Regelabweichung zu ermitteln. Beispielsweise kann die Regelabweichung die Differenz aus Sollwert und Istwert sein, d. h. die Differenz vom gemessenen physiologischen Parameter und dem Zielwert des physiologischen Parameters. Vorzugsweise wird durch die Steuerungseinheit auf Basis der Regelabweichung eine Steuergröße ermittelt, sodass der elektrische Stimulationsreiz derart angepasst wird, um den physiologischen Parameter auf den Zielwert zu führen. Der nunmehr angepasste elektrische Stimulationsreiz geht erneut von den Elektroden aus.

Dabei kann der elektrische Stimulationsreiz derart angepasst werden, dass eine oder mehrere Stimulationsparameter verändert werden. Beispielsweise kann die Stimulationsfrequenz, d. h. die Anzahl von Impulsen innerhalb eines bestimmten Zeitrahmens und/oder die Amplitude des Stimulationsreizes angepasst werden. Vorzugsweise kann die Einstellung der physiologischen Parameter durch die Steuerungseinheit vorgenommen werden. Hierzu liegt auf der Steuerungseinheit bevorzugt eine Software installiert vor, umfassend Algorithmen, um die Anpassung zu ermitteln. Hierbei könnten bevorzugt Algorithmen der künstlichen Intelligenz zum Tragen kommen, welche anhand der Anwendung verschiedener Stimulationsmuster eine optimierte Anpassung der Stimulationsparameter im Hinblick auf die gewünschte Regelung finden. Ebenso kann es bevorzugt sein, dass der Steuereinrichtung Algorithmen installiert sind, welche anhand von Erfahrungswerten oder klinischen Studien eine Anpassung der Stimulationsparameter anhand einer Abweichung des physiologischen Parameters vom Zielwert vornehmen. So kann es bevorzugt sein, dass am Anfang der Therapie (bei einer größeren Differenz zwischen Soll- und Istwert) der Stimulationsreiz eine höhere Intensität aufweist als kurz vor dem Ende der Therapie, wenn insbesondere der physiologische Parameter ausreichend nah an den Zielwert geführt worden ist.

In einer weiteren bevorzugten Ausführungsform ist die Vorrichtung dadurch gekennzeichnet, dass die an eine anatomische Form des Ohres angepasste Oberfläche des Schaltungsträgers und/oder die Positionierung der Elektroden auf der Oberfläche des Schaltungsträgers zur Kontaktierung von Bereichen des Ohres auf Daten eines Abbildungsverfahrens eines Bereiches einer Oberfläche des Ohres und/oder eines Verlaufes von Nervenfasern und/oder Nervenbündel, bevorzugt Nervenfasern und/oder Nervenbündel des Vagusnervs, basiert.

Vorteilhafterweise kann ein Nutzer durch die Anpassung an eine anatomische Form des Ohres die Vorrichtung besonders stabil, langanhaltend und zuverlässig tragen. Insbesondere wird vorteilhaft die Vorrichtung auf die individuelle Gestaltung des Ohres angepasst. Zwar ähneln sich Ohren von Mensch zu Mensch, die tatsächliche anatomische Ausgestaltung und/oder Struktur des Ohres kann jedoch verschieden ausfallen. Daher ist es besonders vorteilhaft, eine individuelle Anpassung der Vorrichtung, insbesondere des Schaltungsträgers, bereitzustellen, mit der in Bezug auf die etwaige individuelle Besonderheit der anatomischen Form des Ohres eines Nutzers der Schaltungsträger umfassend die Elektroden optimal am Ohr aufsitzt.

Im Stand der Technik ist es bekannt, beispielsweise durch einen Gips oder ein gipsähnliches Material eine Negativform eines Ohres oder eines Ohrabschnitts zu bilden, diesen zu begießen und daraufhin eine Positivform einer möglichen Stimulationsvorrichtung bereitzustellen. Dies ist jedoch dahingehend nachteilig, dass kleinste, individuelle Strukturen und/oder Formen am Ohr, vorzugsweise im Millimeterbereich, die von Mensch zu Mensch unterschiedlich sind, nicht gut genug abgebildet werden können. Zudem ist das Verfahren aufwendig und kann nicht auf einfache Weise skaliert werden. Ein weiterer Nachteil ist es, dass die intraindividuelle Positionierung der Nervenfasern, insbesondere des Vagusnervs, bei derartigen Methoden, welche lediglich analog eine Oberfläche des Ohres abbilden, nicht berücksichtigt wird.

Vorteilhafterweise werden die eben aufgeführten Nachteile beseitigt, indem bevorzugt durch ein Abbildungsverfahren die Oberfläche des Ohres und/oder der Verlauf von Nervenfasern, vorzugsweise des Vagusnervs, aufgenommen wird.

Durch die Anwendung eines Abbildungsverfahrens können Formen, Verformungen, Strukturen und/oder Missbildungen am Ohr vorteilhaft berücksichtigt werden, die die Individualität der anatomischen Form eines Ohres auszeichnen. Hierdurch erreicht der Schaltungsträger vorteilhafterweise eine optimale und präzise Form, die sich genauestens für das Ohr des Nutzers eignet. Dies ist insbesondere dahingehend vorteilhaft, dass auch eine langanhaltende und damit auch hohe Behandlungseffizienz erreicht werden kann. Weiterhin erhöht sich der Tragekomfort, was die Compliance (Mitwirkung eines Patienten) und somit den Behandlungserfolg erhöht.

Ein Abbildungsverfahren bezeichnet im Sinne der Erfindung bevorzugt ein Verfahren, das eine digitale Abbildung des Ohres oder von Teilbereichen des Ohres aufnimmt. Vorzugsweise ist die Abbildung ein dreidimensionales Abbild des Ohres. Besonders bevorzugt umfasst das Abbildungsverfahren die Aufnahme von Daten über einer anatomischen Oberfläche eines Ohres oder von Teilbereichen davon und deren Umwandlung in eine digitale Repräsentation, welche an eine Vorrichtung zur additiven Fertigung des Schaltungsträgers weitergegeben werden kann. Vorteilhafterweise kann dann der Schaltungsträger im Anschluss anhand der Daten über die additive Fertigungsvorrichtung erstellt werden.

Das Abbildungsverfahren kann beispielsweise ein dreidimensionales Scannen des Ohres und/oder von Ohrbereichen umfassen. Bevorzugt wird hierbei der die Ohrmuschel, Abschnitte der Ohrmuschel und/oder des äußeren Gehörgangs gescannt. Vorzugsweise liegen nach dem Scannen Daten vor, die Informationen hinsichtlich der anatomischen Ausgestaltung der gescannten Abschnitte liefern. Vorteilhaft kann hierdurch ein besonders genaues Abbild des Ohres genutzt werden, um den Schaltungsträger für die erfindungsgemäße Vorrichtung bereitzustellen. In bevorzugten Ausführungsformen können die Daten unmittelbar nach dem Scannen in eine additive Fertigungsvorrichtung übertragen werden. In weiteren bevorzugten Ausführungsformen können die aufgenommen Daten durch eine dazu geeignete Software bearbeitet werden, beispielsweise um die Position von Blutgefäßen, insbesondere von Nervenzweigen und/oder Nervenbündeln des Vagusnervs, zu berücksichtigen.

Bevorzugt wird zum Scannen des Ohres oder Teilbereichen des Ohres eine Vorrichtung verwendet, die ein dreidimensionales Bild oder ein digitales dreidimensionales Modell (3D-Modell) des Ohres oder von Teilbereichen des Ohres erzeugen kann.

Im Stand der Technik sind hierzu eine Reihe von geeigneten Vorrichtungen bekannt.

Beispielsweise ist aus der WO 2017/062868 A1 eine Vorrichtung bekannt, um insbesondere eine Aufnahme des äußeren Gehörgangs und/oder daran anliegenden Bereichen zu erhalten. Dabei wird ein kompakter 3D-Scanner genutzt, der innerhalb von einigen Sekunden ein genaues digitales 3D-Modell erstellen kann. Hierbei wird bevorzugt die Vorrichtung umfassend einer verformbaren Membran am Ohr und/oder in den äußeren Gehörgang geführt und mit Licht bestrahlt, um kleinste Verformungen der Membran durch individuelle kleinste Prägungen zu erkennen.

Ein weiteres Beispiel ist die Vorrichtung gemäß der US 8,900,128 B2 Diese Vorrichtung umfasst einen sogenannten Otoscanner. Dabei weist der Otoscanner eine Ohrsonde, eine Vielzahl von Tracking- und Beleuchtungssensoren und einen Bildsensor auf. Der Bildsensor ist mit einer Weitwinkellinse optisch verbunden, um eine hinreichende Tiefenschärfe zu gewährleisten und nimmt bevorzugt mit einer hohen (Video)Rate Bilder auf. In Abhängigkeit von einer Sequenz von Bildern, die mittels des Bildsensors aufgenommen werden, wenn das gescannte Ohr durch Laserlicht illuminiert wird, und getrackten Positionen der Ohrsonde, die von Reflektionen der Tracking-Illumination abgeleitet werden, die durch den Tracking-Illuminations-Sensor erfasst wurden, kann ein 3D-Bild des gescannten Ohrs konstruiert werden.

In weiteren bevorzugten Ausführungsformen kann das Abbildungsverfahren auch ein Scannen eines bereits vorhandenen Ohrmodells und/oder einer Ohrabformung umfassen. Die Methode zur Aufnahme eines dreidimensionalen Bildes des Ohres ist besonders einfach in der Durchführung, da aus bereits vorhandene Modelle zurückgegriffen werden kann. Des Weiteren kann hierüber eine Mittelung einer Mehrzahl verschiedener (menschlicher) Ohren (bzw. deren Teilbereiche) berücksichtigt werden.

Besonders bevorzugt umfasst das Abbildungsverfahren auch die Aufnahme des Verlaufs von Nervenfasern und/oder Nervenbündel, insbesondere des Vagusnervs, die sich am Ohr, insbesondere unterhalb der Hautoberfläche des Ohres, befinden.

Eine Nervenfaser bezeichnet bevorzugt eine Einheit umfassend ein Axon und seiner Umhüllung umfassend Gliazellen. Gliazellen haben neben einer Stütz- und/oder Haltefunktion auch einen Einfluss auf die Signalweiterleitung, dem Stoffwechsel und/oder die Immunabwehr. Die Gliazellen unterscheiden sich im peripheren Nervensystem und zentralen Nervensystem. Zudem gibt es Nervenfasern mit und ohne Myelinscheide.

Ein Nervenbündel bezeichnet bevorzugt eine Substruktur, die mehrere Nervenfasern umfasst. Ein Nervenbündel stellt somit ein Bündel umfassend mehrere einzelne Fasern dar. Man spricht bei einem Nervenbündel von einem sogenannten Nervenfaszikel.

Die Aufnahme des Verlaufs von Nervenfasern und/oder Nervenbündel kann direkt oder indirekt erfolgen. Nervenfasern und/oder Nervenbündel weisen in der Regel Durchmesser im Submillimeterbereich auf, sodass eine direkte Ermittlung der Positionierung und/oder des Verlaufs mittels optischer Methoden zwar möglich, jedoch aufwändiger ist. Vorteilhaft kann der Verlauf der Nervenfasern durch eine Bildgebung von Blutgefäßen im Ohr approximiert werden. Oftmals sind die Blutgefäße und Nervenfasern miteinander verflochten bzw. verlaufen nebeneinander. Um lokale Verläufe und/oder Verzweigungen von Nervenfasern und/oder Nervenbündel zu finden, kann es beispielsweise bevorzugt sein, die Ohrmuschel von außen zu durchleuchten. Grünes Licht eignet sich diesbezüglich in besonderem Maße, da die Blutgefäße bei einer derartigen Beleuchtung sich deutlich vom umgebenden Gewebe abgrenzen. Hierdurch können die Blutgefäße besonders gut lokalisiert und sichtbar werden. Andere Bildgebungsverfahren können ebenfalls zum Einsatz kommen, die vorzugsweise in gewissen Maße Nervenfasern und/oder Nervenbündeln unter der Hautoberfläche des Ohres sichtbar machen können, insbesondere durch eine Sichtbarkeit von Blutgefäßen. Die sichtbar gemachten Blutgefäße weisen vorteilhaft auf die Präsenz von Nervenfasern und/oder Nervenbündel hin, sodass die Bildgebung eine bevorzugte Positionierung der Elektroden auf dem Schaltungsträger vorgegeben kann, mit der eine effektive transkutane Stimulation der Nervenfaser(n) ermöglicht wird.

In einer weiteren bevorzugten Ausführungsform ist die Vorrichtung dadurch gekennzeichnet, dass die Vorrichtung in den Gehörgang einsetzbar ist, wobei eine Oberfläche des Schaltungsträgers an einen Bereich des Gehörgangs angepasst ist und die Elektroden bevorzugt einen Bereich der Concha und/oder des äußeren Gehörgangs des Ohres kontaktieren.

Vorteilhafterweise eignet sich die Concha des Ohres besonders gut dazu, um eine Stimulation des Vagusnervs durchzuführen, da es bekannt ist, dass ein hoher Anteil an Nervenfasern unterhalb der entsprechenden Hautoberfläche verläuft. Somit eignet sich die Concha besonders gut, um die Elektroden mit der an der Concha befindlichen Hautoberfläche zu kontaktieren, um ein besonders geeignetes Stimulationssignal zu emittieren. Weiterhin ist die Concha dahingehend besonders vorteilhaft, dass der Schaltungsträger besonders einfach und langanhaltend, insbesondere robust, am Patienten applizierbar ist. Eine Platzierung in den äußeren Gehörgang hat sich dahingehend als besonders vorteilhaft erwiesen, dass der Schaltungsträger besonders kompakt ist bei gleichzeitig stabiler Kontaktierung mit der Hautoberfläche.

In einer weiteren bevorzugten Ausführungsform ist die Vorrichtung dadurch gekennzeichnet, dass die Vorrichtung in die Ohrmuschel einsetzbar ist und eine Oberfläche des Schaltungsträgers an einen Bereich der Ohrmuschel angepasst ist, wobei die Elektroden bevorzugt einen Bereich der Concha, bevorzugt Cymba Concha oder Cavum Concha, des Tragus, der Fossa triangularis, der Scapha, der Anthelix und/oder des Antitragus der Ohrmuschel kontaktieren.

Das Außenohr umfasst zwei Bereiche, nämlich die Pinna (Ohrmuschel) und den äußeren Gehörgang. Die anatomische Grenze zwischen diesen beiden Bereichen ist der Porus acticus externus, der den Eingang zum äußeren Gehörgang bildet. Die Ohrmuschel weist eine Vielzahl von anatomischen Abschnitten auf. Diese Abschnitte sind entscheidend, wenn man die Innervation der Ohrmuschel betrachtet. Dabei bezeichnet die Innervation bevorzugt die funktionelle Versorgung eines Organs, eines Körperteils und/oder eines Gewebes mit Nervengewebe, d. h. Nervenzellen und/oder Nervenfasern.

So ist der Tragus, insbesondere die Innenseite des Tragus, ein Bereich der Ohrmuschel, an dem Anteile von Nervenfasern des Vagusnervs besonders dicht verlaufen. Somit eignen sich diese Nervenanteile besonders gut für eine Aufbringung des elektrischen Stromreizes, insbesondere um den Vagusnerv zu stimulieren.

Vorteilhafterweise können eine Vielzahl von Bereichen des Ohres, insbesondere der Ohrmuschel, mit den Elektroden und/oder dem ersten Sensor verbunden werden. Somit wird eine hohe Menge an Möglichkeiten geschaffen, um verschiedene Nervenfasern zu stimulieren, sodass eine verbesserte Therapieoption ermöglicht werden kann. Insbesondere kann vorteilhaft eine besonders hohe Anzahl an Nervenfasern verwendet werden, um den elektrischen Stimulationsreiz weiterzuleiten, was zusätzlich die Behandlung effizienter gestaltet.

In einer weiteren bevorzugten Ausführungsform ist die Vorrichtung dadurch gekennzeichnet, dass die mindestens drei Elektroden mindestens eine Stimulationselektrode und eine Referenzelektrode umfassen.

Mithilfe der mindestens einen Stimulationselektrode und der Referenzelektrode kann insbesondere in den Bereichen des Ohres, innerhalb derer Nervenfasern verlaufen, eine transkutane Nervenstimulation vorgenommen werden. Hierzu wird bevorzugt zwischen der mindestens einen Stimulationselektrode und der Referenzelektrode ein elektrisches Potential erzeugt.

Vorzugsweise dient die mindestens Stimulationselektrode als Kathode und Referenzelektrode als Anode in dem elektrischen Stromkreis, der während der transkutanen Stimulation geschlossen wird. Bevorzugt kann die Vorrichtung auch mehrere Stimulationselektroden und/oder Referenzelektroden aufweisen.

Die Referenzelektrode und die mindestens eine Stimulationselektrode können eine gleiche oder unterschiedliche Form und/oder Größe aufweisen.

Beispielsweise können die Referenzelektrode und/oder die mindestens eine Stimulationselektrode als Metallelektroden mit kugelförmiger oder flacher kreisförmiger Oberfläche zum Einsatz kommen. Es kann auch bevorzugt sein, dass die Elektroden eine rechteckige und/oder andere polygone Form aufweisen.

Vorzugsweise weist die Referenzelektrode eine höhere Oberfläche auf als die mindestens Stimulationselektrode. Vorteilhafterweise ergibt sich durch die Flächenverhältnisse und/oder die unterschiedliche Form und/oder Struktur der Stimulationselektrode und der Referenzelektrode eine hohe Unempfindlichkeit des Stimulationsreizes gegenüber Behaarung und/oder Befettung an der Hautoberfläche.

In bevorzugten Ausführungsformen umfasst die Vorrichtung eine Vielzahl (beispielsweise 3, 4, 5 oder mehr) von Stimulationselektroden und eine Referenzelektrode.

Die Anordnung umfassend Referenzelektrode und Stimulationselektrode kann derart ausgebildet sein, dass die Elektroden mit der Hautoberfläche der Ohrmuschel und/oder Abschnitten der Ohrmuschel kontaktierbar sind. In einigen Ausführungsformen können die Referenz- und/oder Stimulationselektroden eine gebogene Form aufweisen, um besonders passgenau Abschnitte des Ohres kontaktieren zu können. Ebenso können kreisförmige Elektroden bevorzugt sein, welche eine lokalisierter Stimulation zu ermöglichen.

In weiteren besonders bevorzugten Ausführungsformen wird die als Kathode fungierende mindestens eine Stimulationselektrode direkt an einer Stelle des Ohres kontaktiert, die die höchste subkutane Konzentration des Vagusnervs aufweist, sodass durch den abgegebenen negativen Ladungsüberschuss die darunterliegenden Nervenfasern depolarisiert werden. Die subkutane Konzentration des Vagusnervs bezeichnet bevorzugt die Menge an Nervenanteilen, die sich unterhalb der Hautoberfläche, insbesondere des Ohres, befindet.

Insbesondere durch geeignete Flächenverhältnisse der Elektroden kann eine höhere Stromdichte erzielt werden, sodass die Stromdichte steigt, wodurch auch die Wahrscheinlichkeit steigt, dass Vagusäste depolarisiert werden. Die als Anode fungierende Referenzelektrode wird vorzugsweise in einem Abstand zu der mindestens einen Stimulationselektrode auf einem benachbarten Hautabschnitt oder auf eine gegenüberliegenden Seite der Ohrmuschel angeordnet. Vorzugsweise ist ein zu hoher Abstand zu vermeiden, damit nicht zu viel Körpergewebe vom Strom durchflossen wird und auch keine hohe Stromstärke erreicht wird.

Die Vorrichtung ist dadurch gekennzeichnet, dass die Vorrichtung mindestens 3, 5, 10, 20, 50, 100 oder mehr Elektroden aufweist, wobei die Elektroden als Array angeordnet vorliegen, wobei mittels der Steuerungseinheit die Elektroden einzeln steuerbar sind.

Vorteilhafterweise können durch eine hohe Anzahl an Elektroden verschiedene Bereiche der Hautoberfläche der Ohrmuschel mit Elektroden kontaktiert werden, um weitere Bereiche umfassend Nervenfasern mit einem Stimulationsreiz zu versorgen. Dabei sind mittels der Steuerungseinheit die Elektroden einzeln steuerbar. Vorteilhaft kann die Behandlung zusätzlich optimiert werden, da hierdurch je nach Krankheitszustand und/oder Behandlungssituation bestimmte Abschnitte verstärkt stimuliert und andere wiederum vermindert stimuliert werden können. Insbesondere können vorteilhafterweise Elektroden, die mit bestimmten Abschnitten bzw. Nervenenden kontaktiert sind, nicht angesteuert werden. Bevorzugt ergibt sich hierdurch eine besonders hohe Qualität der Behandlung, da die Steuerungseinheit die Auswahl der angesteuerten Elektroden bestimmen kann, beispielsweise nach einer Auswertung der aufgenommenen physiologischen Parameter durch den ersten Sensor.

Hingegen war es im Stand der Technik üblich, dass beispielsweise die Positionierung der Elektroden von einem Arzt verändert und insbesondere angepasst werden musste. Vorteilhaft werden somit solche Eingriffe vermieden, in dem die Steuerungseinheit dazu konfiguriert ist, eine bestimmte Anzahl an Elektroden und insbesondere Elektroden, die sich in bestimmten Positionen an der Hautoberfläche des Ohres befinden, auszuwählen, um den elektrischen Stimulationsreiz am Ohr aufzubringen. Hierdurch kann ein besonders positiver Einfluss auf die neuroelektrische Qualität und damit auf die Funktion der stimulierten Nervenfasern genommen werden.

Weiterhin ist die Auswahl von mehreren Elektroden dahingehend vorteilhaft, dass auch Einfluss auf mehrere physiologische Parameter genommen werden kann. So kann vorteilhafterweise die Behandlung optimiert werden, da bestimmte Nervenfasern, die unterschiedlich dicht verteilt sind an verschiedenen Abschnitten der Ohrmuschel zur Stimulation unterschiedliche Organen bzw. Krankheitsbilder besonders gut geeignet sind.

So ist bereits aus der Akupunktur bekannt, dass beispielsweise eine Stimulation entlang der Cavum Concha die Herzfrequenz beeinflusst, ein Bereich entlang der Cymba hingegen eher die Nieren. Die Areale der Ohrmuschel können vorzugsweise auch durch eine entsprechende Platzierung der Elektroden zur Aufbringung des elektrischen Stimulationsreizes eingesetzt werden.

In einer weiteren bevorzugten Ausführungsform ist die Vorrichtung dadurch gekennzeichnet, dass die Steuerungseinheit dazu konfiguriert ist, anhand einer Auswertung des gemessenen physiologischen Parameters eine Auswahl von Elektroden zu ermitteln, welche besonders geeignet sind, durch einen elektrischen Stimulationsreiz eine Veränderung des physiologischen Parameters zu bewirken. Die Ausführungsform hat sich zeichnet sich durch ein hohes Adaptionsvermögen aus, bei der bewusst eine Vielzahl von Elektroden zunächst bereitgestellt wird, deren tatsächliche Verwendung zur Stimulation des Vagusnerven jedoch von einem messbaren (positiven) therapeutischen Einfluss abhängig gemacht werden kann.

Als besonders vorteilhaft hat es sich herausgestellt, dass die Steuerungseinheit dazu konfiguriert ist, die Auswahl an Elektroden zur Emission eines Stimulationsreizes in Abhängigkeit der Messung des physiologischen Parameters zu treffen. Vorzugsweise kann beispielsweise mittels eines Elektrodenarrays umfassend eine Vielzahl von Elektroden eine transkutane Stimulation an einer Vielzahl von Punkten auf der Oberfläche des Schaltungsträgers vorgenommen werden.

Durch eine Variation der Ansteuerung der Elektroden - unter Überwachung eines physiologischen Parameters - können hierdurch gezielt Elektroden angesteuert werden, welche einen messbaren (positiven) Einfluss auf eine Veränderung des physiologischen Parameters haben. Stimulationssignale an Elektroden, welche eine gewünschte Wirkung auf den physiologischen Parameter erzielen, werden verstärkt, während eine Stimulation durch Elektroden, die keinen oder keinen gewünschten Einfluss auf den physiologischen Parameter haben, vermindert oder beendet werden.

Vorteilhafterweise können hierdurch eine optimale Verteilung eines räumlichen Stimulationsmusters an Nervenfasern und/oder Nervenbündeln erzeugt werden, welche eine Therapie bzw. Heilungsprozess und/oder ein individuelles Wohlbefinden beschleunigt und/oder intensiviert herbeiführen.

Zur Auswahl und zur Ansteuerung der Elektroden, mit denen eine optimierte Stimulation erreicht werden kann, können nicht nur statistische Analysen oder modellbasierte Berechnungen, sondern bevorzugt auch Algorithmen der künstlichen Intelligenz zum Einsatz kommen. Hierbei werden insbesondere Rechenschritte und/oder Handlungsvorschriften ausgeführt, die auf Methoden der künstlichen Intelligenz basieren. Vorzugsweise liegt eine Software oder Computerprogramm auf der Steuerungseinheit installiert vor, welches Befehle zur Ausführung der Rechenschritte und/oder Handlungsvorschriften umfasst.

In einer weiteren bevorzugten Ausführungsform ist die Vorrichtung dadurch gekennzeichnet, dass die Steuerungseinheit dazu konfiguriert ist, mit Algorithmen der künstlichen Intelligenz, bevorzugt mit Machine-Learning Algorithmen umfassend Supervised Learning, Unsupervised Learning und/oder Reinforcement Learning, eine Auswahl von Elektroden zu ermitteln, welche besonders geeignet sind, durch einen elektrischen Stimulationsreiz eine Veränderung des physiologischen Parameters zu bewirken. Bevorzugt können die Algorithmen auch eingesetzt werden, um einen geeigneten Stimulationsreiz für den Patienten zu ermitteln.

Die künstliche Intelligenz wird hierbei als Oberbegriff eingesetzt, der mehrere Technologien umfasst, beispielsweise das Machine-Learning. Im Sinne der Erfindung bezeichnen Machine Learning Algorithmen einen Teilbereich der künstlichen Intelligenz (Artificial Intelligence). Machine Learning nutzt dabei mathematische und statistische Modelle, um aus Datenbeständen zu "lernen". Im Allgemeinen haben Machine Learning Algorithmen den Vorteil, dass Informationen, die für einen menschlichen Beobachter zu komplex sind, automatisch aus einem großen Datensatz extrahiert werden können. Es gibt eine Vielzahl von Machine Learning Algorithmen, die sich im Wesentlichen in drei unterschiedliche Lernverfahren unterscheiden lassen: Supervised Learning, Unsupervised Learning und Reinforcement Learning.

In einer bevorzugten Ausführungsform wird ein Supervised Learning für die Analyse oder Verarbeitung der aufgenommenen physiologischen Parameter genutzt. Beim Supervised Learning Verfahren wird zunächst ein sogenannter Trainingsprozess durchgeführt. Hierbei werden Trainingsdaten in Form von Eingabedaten zusammen mit den entsprechenden Zieldaten bereitgestellt. Der Zweck eines Trainings ist allgemein in Machine Learning Verfahren, Parameter einer Funktion so anzupassen, dass die Funktion anschließend in der Lage ist, den Zielwert mit hoher Genauigkeit von dem entsprechenden Eingabewert zu bestimmen. Die angepasste Funktion wird dann nach dem Trainingsprozess zur Vorhersage von Zieldaten für zuvor nicht sichtbare Eingabedaten verwendet. Die Funktion wird dabei durch ein mathematisches und/oder statistisches Modell beschrieben.

In einer bevorzugten Ausführungsform wird hierbei die Funktion durch Support Vektor Machine, Bayes Netze und/oder Entscheidungsbäume ausgestaltet. Besonders bevorzugt wird die Funktion durch ein künstliches neuronales Netz beschrieben. Hierbei können die künstlichen neuronalen Netze erfindungsgemäß verschiedene Architekturen aufweisen. Im Kontext der Erfindung sind die Eingabedaten bevorzugt durch aufgenommene physiologische Parameter bestimmt.

In einer weiteren bevorzugten Ausführungsform wird das Verfahren des Unsupervised Learning für die Analyse und/oder Verarbeitung der aufgenommenen physiologischen Parameter genutzt. Beim Unsupervised Learning versucht der Algorithmus in den Eingabedaten Muster zu erkennen, die von einem strukturlosen Rauschen abweichen. Die Funktion im Trainingsprozess orientiert sich dabei nur an den Ähnlichkeiten der Eingabedaten und passt ihre Parameter dementsprechend an, sodass keine Ausgabedaten für den Trainingsprozess verwendet werden.

In einer bevorzugten Ausführungsform werden anhand des Unsupervised Learning Verfahrens Segmentierungen bzw. Clustering der Eingabedaten oder aber auch bevorzugt Komprimierungen der Eingabedaten durchgeführt.

In einer bevorzugten Ausführungsform umfasst der Unsupervised Learning Algorithmus bevorzugt die Hauptkomponentenanalyse (Principal Component Analysis (PCA)) und/oder den KMeans- Algorithmus und/oder mindestens ein neuronales Netz.

Wie bereits beschrieben, werden in beiden oben benannten Verfahren in einem ersten Schritt sogenannte Trainingsprozesse zum Ermitteln von optimalen Parametern einer o. g. Machine Learning Funktion durchgeführt. Anhand der angepassten Funktion werden nach dem Training verschiedene Aussagen für vorher unbekannte Eingabedaten getätigt.

In einer weiteren bevorzugten Variante wird das Verfahren des Reinforcement Learning für die Analyse oder Verarbeitung der aufgenommenen physiologischen Parameter genutzt. Beim Reinforcement Learning (Lernen durch Verstärkung) Verfahren findet hingegen der Trainingsprozess kontinuierlich auch nach Anpassung der Parameter einer Funktion statt. Über "Trial and Error" werden Auswirkungen verschiedener Aussagen anhand der angepassten Funktion für bisher unbekannte Eingabedaten beobachtet und bewertet. Als Reaktion auf diese Aussagen erhält der Algorithmus ein Feedback, abstrakt dargestellt in Form einer Belohnung oder Bestrafung, woraufhin der Algorithmus die Funktion anhand ihrer Parameter weiter optimiert. Dementsprechend passt der Algorithmus die Funktion des Machine Learning Verfahrens kontinuierlich an bzw. verändert diesen. Bevorzugt kann das Reinforcement Learning die Q-Learning Methode und/oder o. g. neuronale Netze und/oder weitere neuronale Netze sowie weitere für den Fachmann bekannte Algorithmen nutzen.

Physiologische Parameter bezeichnen im erfindungsgemäßen Kontext bevorzugt Parameter, die Funktionen des menschlichen Körpers widerspiegeln. Hierzu gehören beispielsweise eine Körpertemperatur, eine Pulsrate, ein Blutdruck, eine Sauerstoffsättigung, ein pH-Wert der Haut oder eine Hautleitfähigkeit. Mithilfe von physiologischen Parametern können bevorzugt Schlüsse hinsichtlich des Wohlbefindens und/oder Gesundheitszustand eines Nutzers gezogen werden. Vorzugsweise ist der mindestens eine erste Sensor dazu eingerichtet, einen oder mehrere physiologische Parameter eines Trägers der Vorrichtung zu messen.

In einer weiteren bevorzugten Ausführungsform ist die Vorrichtung dadurch gekennzeichnet, dass der mindestens eine erste Sensor ausgewählt ist aus einer Gruppe umfassend ein Temperatursensor, ein Bewegungssensor, ein Pulssensor, ein Blutdrucksensor, ein Blutflusssensor, einen Pulsoximetriesensor und/oder ein pH-Sensor.

Vorteilhafterweise haben sich die Sensoren als besonders geeignet etabliert, um eine Vielzahl von möglichen physiologischen Parameter zuverlässig zu messen und die aufgenommenen physiologischen Parameter an die Steuerungseinheit zu übertragen. Insbesondere eignen sich die aufgezählten Sensoren dahingehend besonders gut, um fundierte Informationen über den Gesundheitszustand und/oder den Therapiefortschritt eines Nutzers zu erhalten.

Ein Temperatursensor bezeichnet einen Sensor, der dazu konfiguriert ist, die Körpertemperatur des Nutzers zu messen. Dabei kann die durchschnittliche Körpertemperatur und/oder die Körpertemperatur von gewissen Bereichen des Körpers aufgenommen werden. Die Regulation der Körpertemperatur gehört zu den existentiellen Funktionen des Menschen. Die Körpertemperatur ist ein Vitalparameter wie die Atemfrequenz, die Herzfrequenz und/oder der Blutdruck. Der Temperatursensor kann bevorzugt mit elektronischen Messmethoden und/oder auf Basis von Wärmestrahlung die Temperatur messen. Bei elektronischen Messmethoden werden bevorzugt Thermistoren und/oder Thermoelemente eingesetzt, die ein besonders gut wärmeleitendes Material aufweisen und sich insbesondere durch eine Messgenauigkeit von ±0,1 °C auszeichnen. Bei Methoden auf Prinzip der Wärmestrahlung wird vorteilhaft kein direkter Kontakt mit der Hautoberfläche benötigt. Beispielsweise kann unter Verwendung von Infrarottechnologie von der Temperatur des Trommelfells oder der Temporalarterie auf die Körpertemperatur geschlossen werden.

Insbesondere die Körpertemperatur ist ein besonderer Faktor für eine Ermittlung des Gesundheitszustandes und/oder Wohlbefindens. Abweichungen vom individuellen Sollwert der Körpertemperatur können Funktionsstörungen zur Folge haben, die auch die allgemeine körperliche und/oder geistige Leistungsfähigkeit einschränken. So können auch Stress, entzündliche Krankheitsprozesse, Stoffwechselstörungen und/oder Bluthochdruck zu einer erhöhten Körpertemperatur führen. Vorteilhaft kann die Körpertemperatur als Hinweis für eine Vielzahl von physischen und/oder psychischen Belastungen gemessen, ausgewertet und daraufhin ein Stimulationsmuster angepasst werden.

Ein Bewegungssensor bezeichnet einen Sensor, der dazu konfiguriert ist, eine Aktivität, insbesondere eine Bewegung des Nutzers, vorzugsweise auch Bewegungen von bestimmten Körperabschnitten, zu erkennen. Dies ist insbesondere dahingehend relevant, dass Bewegungen des Patienten sich auf einige physiologische Parameter auswirken können, beispielsweise ein erhöhter Puls bei sportlichen Aktivitäten. Vorzugsweise beruht der Bewegungssensor auf einen Beschleunigungssensor, sodass körperliche Bewegungen innerhalb eines Zeitrahmens erfasst werden können. Insbesondere kann die Beschleunigung dort gemessen werden, wo der Sensor befestigt ist. Vorteilhaft kann der Bewegungssensor bevorzugt auch Tagesprofile zur körperlichen Aktivität, den Energieverbrauch, die Schrittzahl, zurückgelegte Distanzen und/oder Höhen messen. Durch einen Bewegungssensor können insbesondere auch Körperschwankungen als ein physiologischer Parameter festgestellt werden, welcher in Bezug auf Krankheiten im Zusammenhang mit Gleichgewichtsstörungen einen relevanten Indikator für den Gesundheitszustand und/oder therapeutischen Fortschritt darstellen kann.

In weiteren bevorzugten Ausführungsformen ist der Bewegungssensor zudem von Relevanz, wenn sich der einem Feedback zugrunde gelegte physiologische Parameter unabhängig von der Nervenstimulation verändert. Beispielsweise kann durch den Bewegungssensor sportliche Aktivitäten ermittelt werden, welche einen Einfluss auf physiologische Parameter wie die Herzfrequenz haben können. Mithilfe des Bewegungssensor können derartige Aktivitäten bei der Feedbackregelung erkannt und berücksichtigt werden. Beispielsweise kann verhindert werden, dass durch einen geschlossenen Regelkreis Stimulationsreize emittiert werden, welche es anstreben einen durch die sportliche Aktivität erhöhten Puls auf einen Ruhepuls zuführen.

Ein Pulssensor ist ein Sensor, der dazu eingerichtet ist, den Puls des Nutzers zu messen. Als Puls bezeichnet man sowohl die Frequenz der beim Pulszählen erfassbaren Druckstöße (Anzahl pro Minute) als auch deren Amplitude und Verlauf (Pulsqualität, z. B. "weicher", "schwacher" oder "schwirrender" Puls). Im Gefäßsystem breitet sich der Puls als Welle aus mit lokalen Zeitverläufen jeweils des Drucks, der Querschnittsfläche, des Volumenstroms und/oder der Strömungsgeschwindigkeit. Insbesondere ist der Pulssensor in der Lage, die Herzfrequenz zu bestimmen. Die Herzfrequenz bezeichnet die Anzahl der Herzschläge pro Zeitintervall, beispielsweise Herzschläge pro Minute. Auch der Puls stellt einen vorteilhaften physiologischen Parameter dar, welcher Rückschlüsse auf einen physischen und/oder psychischen Zustand eines Nutzers erlaubt bzw. welcher als Grundlage für eine Feedbackregulation zur Beeinflussung selbiger durch eine VNS dienen kann. Beispielsweise kann ein erhöhter Puls ein Hinweis für eine Stresssituation sein, sodass die Vorrichtung durch entsprechende Anpassung der elektrischen Stimulationssignale auf eine Stressreduktion hinwirken kann.

Ein Blutdrucksensor betrifft bevorzugt einen Sensor, der der Messung des Blutdruckes dient. Dieser ist insbesondere ein Drucksensor, der bevorzugt über den arteriellen Verlauf des Blutes den Druck messen kann. Die Messung ist genau und bietet den Vorteil einer kontinuierlichen Überwachung. Der Blutdruck kann ebenfalls ein wichtiger Indikator sein, um einen Hinweis auf ein optimales Behandlungsregime zu erhalten. So ist ein hoher Blutdruck gefährlich für die Arterien und begünstigt die Entstehung von Krankheiten. Die Folgen eines hohen Blutdruckes können Herzinfarkt, Schlaganfall und/oder Durchblutungsstörungen in den Nieren oder den Beinen sein. Niedriger Blutdruck können Schwäche- und/oder Schwindelgefühle auftreten, insbesondere auch Ohnmacht. Vorteilhafterweise kann durch die Überwachung des Blutdruckes und Anpassung der Stimulationsreize für eine positive Einflussnahme vorgenannten Gesundheitsstörungen besonders effektiv behandelt oder vorgebeugt werden.

Ein Blutflusssensor bezeichnet bevorzugt einen Sensor, der insbesondere die Strömungsgeschwindigkeit des Blutes bestimmen kann. Vorzugsweise wird Licht und/oder Schall emittiert und insbesondere unter Ausnutzung des Doppler-Effektes die Fließgeschwindigkeit bestimmt. Dabei sendet ein Sender eine Welle mit einer bestimmten Frequenz aus. Die Welle durchdringt die Haut und trifft auf ein rotes Blutkörperchen, das sich vom Sender wegbewegt. Die vom bewegten Blutkörperchen gestreute Welle wird von einem Empfänger registriert. Auf Basis der Sende- und Empfangsfrequenz kann daraufhin die Strömungsgeschwindigkeit bestimmt werden. Durch einen erhöhten Blutfluss zirkuliert das Blut schneller durch den Körper. Dadurch werden die Gefäße gedehnt und der Blutstrom reizt die Gefäßwände. Diese Dehnung löst einen chemischen Reiz in der Gefäßwand aus, der Alterungsprozessen der Gefäße entgegenwirkt und diese elastischer hält. Vorteilhaft kann durch die erfindungsgemäße Vorrichtung durch Emission des Stimulationsreizes auch ein Blutfluss als Regulationsgröße genutzt werden, um den Gesundheitszustand und/oder das Wohlbefinden zu steigern.

Ein Pulsoximetriesensor bezeichnet einen Sensor, der dazu eingerichtet ist, die Sauerstoffsättigung im Blut zu messen. Vorzugsweise erfolgt die Messung durch Lichtabsorption bzw. Lichtremission bei Durchleuchtung der Haut (perkutan). Das Pulsoxymeter stellt ein speziell auf diese Anwendung optimiertes Spektralphotometer dar. Die Sauerstoffsättigung gibt an, wie viel Prozent des gesamten Hämoglobins im Blut mit Sauerstoff beladen sind Sie liefert somit Informationen über die Effektivität des Sauerstofftransportes und damit auch über die Atmung. Das Messprinzip basiert auf dem Umstand, dass mit Sauerstoff beladenes Hämoglobin, sogenanntes oxygeniertes Hämoglobin (HbO₂), bei optischen Wellenlängen einen deutlich anderen Absorptionsverlauf als desoxygeniertes Hämoglobin (Hb) - also Hämoglobin, dessen Transportplätze für O₂ noch frei sind - aufweist. Die Sauerstoffsättigung gibt somit Hinweise hinsichtlich der Atmung oder der Lungenfunktion eines Nutzers. Somit kann durch die erfindungsgemäße Vorrichtung besonders zuverlässig die Atmungsfunktion durch eine Messung der Sauerstoffsättigung überwacht werden. Bei von der Norm abweichenden Messwerten kann die Emission elektrischer Stimulationsreize angepasst werden, um dem entgegenzuwirken und den Sauerstoffgehalt und damit auch die Atemfunktion auf einen gewünschten Zielwert hinzuführen.

Ein pH-Sensor bezeichnet einen Sensor, der den pH-Wert der Haut messen kann. Dazu verfügt der Sensor über eine Elektrode, die mit der Haut kontaktiert wird, um den pH-Wert zu bestimmen. Der pH-Sensor ist bevorzugt mit einer flachen Elektrode ausgestattet, um Messungen direkt auf der Kontaktfläche zu ermöglichen. Vorzugsweise wird als Elektrode ein Diaphragma eingesetzt, um einen direkten Kontakt mit minimaler Feuchtigkeit und hoher Stabilität zu bieten. Typischerweise ist der pH-Wert der menschlichen Haut im sauren Bereich. Wenn der pH-Wert der Haut in den alkalischen Bereich steigt, stört dies das natürliche Gleichgewicht. Wichtige Hautfette (Lipide) können nicht mehr aufgebaut werden, sodass die Haut Wasser verliert und trocken werden kann. Die Messung des pH-Wertes ist mithin beispielsweise relevant, wenn der Nutzer schwitzt. Eine Messung kann beispielsweise auch in Wechselwirkung mit dem Bewegungssensor, dem Temperatursensor und/oder dem zweiten Sensor erfolgen, um beispielsweise deutlich feststellen zu können, wodurch und/oder wie eine Veränderung des pH-Wertes herbeigeführt wurde und/oder durch eine entsprechende Anpassung der Nervenstimulation auf eine positive Entwicklung hinzuwirken. Darüber hinaus ist die Messung des pH-Wertes der Haut auch dahingehend relevant, dass der pH-Wert ein Indikator für den Halt der Vorrichtung am Ohr darstellen kann. Eine zu trockene Haut kann sich nachteilig auf den Halt der Vorrichtung am Ohr auswirken. Vorteilhafterweise kann somit erkannt werden, wann die notwendige Stabilität am Ohr nicht mehr vorliegt.

In einer weiteren bevorzugten Ausführungsform ist die Vorrichtung dadurch gekennzeichnet, dass die Vorrichtung einen zweiten Sensor umfasst, wobei der zweite Sensor dazu konfiguriert ist, Umweltparameter zu messen, wobei bevorzugt die Umweltparameter ausgewählt sind aus einer Gruppe umfassend eine Umgebungstemperatur, einen Luftdruck und/oder eine Luftfeuchtigkeit.

Vorteilhafterweise werden durch den zweiten Sensor auch Umwelteinflüsse beachtet, die durchaus physiologische Parameter, insbesondere Vitalparameter, beeinflussen können. So ist es hinlänglich bekannt, dass bei erhöhter Umgebungstemperatur auch die Körpertemperatur ansteigen kann, was wiederrum zur erhöhten Schweißbildung führt, was eine mögliche Änderung des pH-Wertes der Haut zur Folge hat. Ebenso ist bekannt, dass eine erhöhte Außentemperatur und/oder ein verringerter Luftdruck die Atemfrequenz und damit auch den Puls und/oder die Sauerstoffsättigung beeinflusst. Vorteilhafterweise können durch den zweiten Sensor äußere Einflüsse, insbesondere Umweltparameter, erfasst werden und bei der Behandlung des Probanden berücksichtigt werden, was die Qualität der Behandlung deutlich erhöht.

Im Sinne der Erfindung bezeichnet ein Umweltparameter bevorzugt einen Parameter, der insbesondere zur Beschreibung der Umgebung dient, in der sich ein Nutzer befindet. Dies kann beispielsweise, jedoch nicht darauf beschränkt, durch die Umgebungstemperatur, die Luftfeuchtigkeit, der Luftdruck, der Lärmpegel und/oder die Schadstoffbelastung gegeben sein.

Ein Umwelteinfluss bezeichnet bevorzugt den Einfluss, den ein oder mehrere Umweltparameter auf den Patienten haben können.

In einer weiteren bevorzugten Ausführungsform ist die Vorrichtung dadurch gekennzeichnet, dass die Steuerungseinheit dazu konfiguriert ist, eine Messung von Umweltparametern für die Anpassung der Stimulationsparameter für den elektrischen Stimulationsreiz zu berücksichtigen.

Hierzu werden vorzugsweise Umweltparameter, die von dem zweiten Sensor aufgenommen werden, an die Steuerungseinheit übermittelt. Die Steuerungseinheit ist bevorzugt derart konfiguriert, dass Umwelteinflüsse nicht als Wirkung der Stimulation aufgefasst werden. Stattdessen können die Umweltparameter dazu dienen, Änderungen physiologischer Parameter, welche nicht mit der Stimulation kausal in Zusammenhang stehen, korrekt zu interpretieren. Hierdurch kann vorteilhafterweise die Zuverlässigkeit der Behandlung nochmals erhöht werden. Beispielsweise kann die Erhöhung des Pulses durch eine erhöhte Umwelttemperatur und nicht durch die Nervenstimulation resultieren. Vorteilhafterweise kann durch die Wechselwirkung der Steuerungseinrichtung mit dem ersten Sensor und/oder dem zweiten Sensor erkannt werden, wodurch und welche physiologischen Parameter durch die Behandlung und/oder durch Umwelteinflüsse verändert werden.

In bevorzugten Ausführungsformen werden Umwelteinflüsse im geschlossenen Regelkreis berücksichtigt. Dabei können Umwelteinflüsse im Zusammenhang mit dem geschlossenen Regelkreis als sogenannte Störgröße aufgefasst werden. Einfallende Störgrößen, insbesondere in Form von Umwelteinflüssen, werden beispielsweise durch den Vergleich zwischen den aufgenommen physiologischen Werte, den zu erreichenden Zielwerten und/oder den Umweltparametern berücksichtigt.

Der geschlossene Regelkreis wird bevorzugt durch das Einwirken der Störgröße auf die physiologischen Parameter und/oder Zielwerten angepasst. So kann vorteilhafterweise der Zielwert optimiert werden, da beispielsweise bei hohen Umgebungstemperaturen ein anderer Zielwert benötigt werden kann als bei niedrigen Zielwerten. Vorteilhaft kann auch der dazu notwendige dafür aufzubringende elektrische Stimulationsreiz durch eine Optimierung von Stimulationsparametern angepasst werden.

Vorteilhafterweise bringt die Aufnahme, insbesondere die Berücksichtigung, physiologischer Parameter und Umweltparameter bei der Aufbringung des elektrischen Stimulationsreizes einen Synergieeffekt hervor, da ein besonders geeigneter und optimaler Stimulationsreiz auf deren Grundlage von der Steuerungseinheit erstellt wird.

Weiterhin erweitern sich durch die bevorzugte Ausführungsform ein Verwendungsbereich der Vorrichtung auf ungewöhnliche bzw. außeralltägliche Situationen. So kann beispielsweise der Nutzer auch während einer Bergsteigung die Vorrichtung tragen, bei der mit zunehmender Höhe der Luftdruck und/oder die Temperatur sinkt, sodass der Normwert für Sauerstoffsättigung, Puls etc. abweichen kann. Solche Aktivitäten wirken sich vorteilhaft nicht beeinträchtigend auf die optimierte Stimulation während der Behandlung aus, da durch den geschlossenen Regelkreis die veränderten Umweltparameter berücksichtigt werden können. Die erfindungsgemäße Vorrichtung eignet sich somit bei einer Vielzahl von Aktivitäten, in der stets ein auf den Nutzer und auch im Hinblick von Umgebungsparametern optimaler und effektiver elektrischer Stimulationsreiz aufgebracht werden kann.

In einer weiteren bevorzugten Ausführungsform ist die Vorrichtung dadurch gekennzeichnet, dass die Vorrichtung eine Kommunikationseinheit zur Verbindung mit einer externen Datenverarbeitungseinheit und/oder einem Eingabemodul umfasst.

Vorteilhafterweise können die aufgenommen physiologischen und/oder Umweltparameter durch die Kommunikationseinheit an eine externe Datenverarbeitungseinheit übertragen werden. Hierdurch kann beispielsweise die externe Datenverarbeitungseinheit eine zusätzliche Analyse von aufgenommenen Daten durchführen. Dies kann insbesondere dann gemacht werden, wenn Kontrolluntersuchungen beim Patienten notwendig sind.

Darüber hinaus kann es bevorzugt sein, dass durch eine Eingabe von bestimmten Stimulationsparametern an einem Eingabemodul ein elektrischer Stimulationsreiz am Patienten aufgebracht wird. Beispielsweise kann der Arzt während Kontrolluntersuchungen eine zusätzliche Stimulation ausgehend vom Eingabemoduls vornehmen, um neben der bereits durchgeführten Stimulation eine aus seiner Sicht geeignetere Stimulation durchzuführen. Dies kann sich vorteilhaft auf die Behandlungseffizienz auswirken.

Durch die Kommunikationseinheit kann vorzugsweise die Übertragung der Daten des Eingabemoduls kabellos erfolgen. Vorteilhafterweise vermindert dies den Aufwand, um zwischen der Kommunikationseinheit und dem Eingabemodul eine Datenverbindung herzustellen.

Es kann auch bevorzugt sein, dass die Vorrichtung Anschlussstellen aufweist, die dazu geeignet sind, mit entsprechenden Kabeln ausgehend vom Eingabemodul angeschlossen zu werden, um daraufhin mit einer externen Quelle die Stimulation auszuführen. Beispielsweise kann ausgehend vom Eingabemodul der elektrische Stimulationsreiz an die Vorrichtung übertragen und auf den Patienten aufgebracht werden.

Eine Kommunikationseinheit bezeichnet im Sinne der Erfindung bevorzugt eine Einrichtung zum Übertragen, insbesondere zum Senden und/oder Empfangen, von Daten. Die Übertragung erfolgt bevorzugt durch gerichtete oder ungerichtete elektromagnetische Wellen, wobei der Bereich des genutzten Frequenzbands je nach Anwendung und verwendeter Technik von wenigen Hertz (Niederfrequenz) bis hin zu mehreren hundert Terahertz variieren kann, wobei beispielsweise folgende Datenübertragungsverfahren genutzt werden können: Bluetooth, WLAN, ZigBee, NFC, Wibree oder WiMAX im Radiofrequenzbereich sowie IrDA und optischer Richtfunk (FSO) im infraroten bzw. optischen Frequenzbereich.

Im Sinne der Erfindung bezeichnet ein Eingabemodul bevorzugt eine Vorrichtung, auf der insbesondere Informationen, wie beispielsweise Stimulationsparameter, eingegeben werden können. Die eingegebenen Informationen können dann weiterverarbeitet werden. Im Kontext der Erfindung ist es bevorzugt, dass Stimulationsparameter in das Eingabemodul eingegeben werden können und diese dann an die Vorrichtung übertragen werden. Beispielsweise kann das Eingabemodul ein Computer, ein Touchpad, ein Smartphone und/oder eine sonstige Vorrichtung sein, die dazu geeignet ist, Informationen zu erhalten und/oder auszusenden. Insbesondere ist das Eingabemodul dazu eingerichtet, sich mit dem Kommunikationsmittel zu verbinden.

In einer weiteren bevorzugten Ausführungsform ist die Vorrichtung dadurch gekennzeichnet, dass das elektrische Signal durch einen Gleichstrom oder einen Wechselstrom bereitgestellt wird, wobei bevorzugt im Falle eines Wechselstroms eine Frequenz zwischen 0,1 - 100 Hz vorliegt, wobei bei Wechselstrom bevorzugt eine konstante Impulsfolge, eine unterbrochene Impulsfolge und/oder ein Rechtecksignal eingesetzt wird.

Die Selektivität bei der Stimulation von jeweiligen Nervenfasern, wie beispielsweise Aβ- und Aδ-Fasern, hängt in hohem Maße vom Stimulationsmuster ab. Vorzugsweise wird eine Frequenz von ca. 20 - 25 Hz für die periphere elektrische Stimulation des Parasympathikus eingesetzt. Niedrige Frequenzen von ca. 0,5 - 10 Hz sind für das sympathische System besonders gut geeignet sind.

Hohe Frequenzen haben eine kurze depolarisierende Halbwertszeit und sind daher in der Lage, leicht erregbare dicke Nervenfasern zu stimulieren, wie beispielsweise myeliniserte Aß-Fasern, die indirekt das parasympathische System aktivieren können.

Im Gegensatz dazu sind breite depolarisierende Perioden mit niedrigen Frequenzen erforderlich, um dünne Nervenfasern, wie z. B. myelinisierte Aδ- oder nicht-myelinisierte C-Fasern, zu stimulieren, die in der Regel das sympathische System behandeln.

Um den Vagusnerv und/oder Nervenfasern des Vagusnervs zu stimulieren, können beispielsweise monophasische Rechteckimpulse etwa alle 0,5 bis 5 Sekunden (1-Hz-Stimulation) mit wechselnder Polarität und einer Impulsbreite von ca. 0,1 ms bis 10 m, eingesetzt. In bevorzugten Ausführungsformen bestimmt die Pulslänge die Art der zu stimulierenden Fasern. Das heißt, kurze Pulse stimulieren im Wesentlichen leicht erregbare dicke Fasern, während lange Pulse sowohl dicke als auch dünne Fasern stimulieren können.

In einer bevorzugten Ausführungsform werden zur Stimulation oszillierende elektrische Signale aufgebracht. Dazu kann die Vorrichtung einen Oszillator aufweisen. Im Rahmen einer Logik- und/oder Steuerschaltung, die bevorzugt durch eine elektronische Schaltung bereitgestellt wird, erfolgt die Verarbeitung der aufgenommenen und aufzubringenden Stimulationsreize. Weiterhin kann die Vorrichtung elektronische Schaltungen und/oder Komponenten aufweisen, die für die Anpassung der Stimulationsparameter des Stimulationssignales eingerichtet sind. Beispielsweise können Stimulationsstärkeregler zur Regelung der Amplitude des Stimulationssignales vorgesehen sein. Hohe Amplituden führen bevorzugt zu einer höheren Stimulation, insbesondere des Vagusnervs, als niedrigere Amplituden. Die Amplitude des Stroms kann je nach Patient sowie zu behandelnder Krankheit bzw. gewünschten Behandlungserfolg interindividuell verschieden angepasst werden. Bevorzugt erfolgt die Anpassung durch den geschlossenen Regelkreis. Ferner kann auch ein Stimulationsfrequenzregler zur Regelung des Frequenzmusters des Stimulationsreizes vorhanden sein. So können beispielsweise schnell aufeinander folgende Stimulationsreize aufgebracht werden oder Stimulationsreize, die mit einem größeren Abstand nacheinander emittiert werden.

Die Amplitude, insbesondere die Stromstärke, hängt bevorzugt davon ab, bei welchen Frequenzen der Stimulationsreiz emittiert/oder davon, ob Wechselstrom oder Gleichstrom eingesetzt und auch von der Stimulationszeit (Dauer eines aufgebrachten Stimulationssignales) und der Kontaktfläche zwischen den Elektroden und der Haut. wird. Die Stromstärke kann beispielsweise bevorzugt zwischen ca. 1 mA - 30 mA oderzwischen ca. 10 mA - 30 mA liegen.

Im Sinne der Erfindung bezeichnet ein elektrischer Stimulationsreiz bzw. ein elektrisches Stimulationssignal bevorzugt einen zeitlichen Verlauf eines Stroms, welche durch die Haut gelangt und Nervenfasern stimuliert. Eine Stimulation meint vorzugsweise, dass die Nervenfasern angeregt werden. Die Stimulation kann beispielsweise derart erfolgen, dass die Anregung oder die Hemmung von bestimmten Botenstoffen resultiert und/oder Fehlfunktionen an der Synapse, der Stelle des Informationsaustausches zwischen Nervenzellen (Neuronen), behoben werden. Insbesondere bei Krankheiten wie Demenz und/oder Parkinson liegen Beeinträchtigungen an den Synapsen vor.

Ein elektrischer Impuls meint vorzugsweise einen elektrischen Strom, der zeitlich limitiert ist. Insbesondere wird bei einem elektrischen Impuls ein elektrischer Strom innerhalb eines bestimmten Zeitbereichs ausgesendet.

Ein Stimulationsmuster bezeichnet bevorzugt einen elektrischen Stimulationsreiz, der mit bestimmten Stimulationsparametern kontinuierlich und/oder diskret, ausgehend von den Elektroden, emittiert wird. Ein diskretes Stimulationsmuster wäre beispielsweise die Emission einer Mehrzahl von aufeinanderfolgenden elektrischen Impulsen. Insbesondere kann ein Stimulationsmuster ein zeitlich wiederkehrender, regelmäßiger Stimulationsreiz sein.

Aδ-Fasern sind eine Klasse dünn myelinisierter Nervenfasern. Sie dienen hauptsächlich der Temperaturempfindung sowie der schnellen Schmerzleitung. Ihr Durchmesser wird im Schnitt mit ca. 2 - 5 µm angegeben. Ihre Nervenleitgeschwindigkeit beträgt etwa 10 - 30 m/s.

Aß-Fasern bilden ebenfalls eine Klasse myelinisierter Nervenfasern. Sie leiten Signale des Tastsinns bzw. der kutanen Mechanosensorik weiter, d. h. Berührungen, Drücke und/oder Vibrationen. Aß-Fasern haben einen Durchmesser von ca. 5 - 15 µm und eine Nervenleitgeschwindigkeit von ca. 30 - 75 m/s.

C-Fasern sind langsam leitende, unmyelinisierte Nervenfasern. Sie dienen der afferenten Erregungsleitung. Sie haben einen Durchmesser von ca. 0,2 - 1,5 µm. Im Gegensatz zu vielen anderen Nervenfasern sind sie nicht myelinisiert, d. h. sie weisen keine Markscheide auf. Aus diesem Grund beträgt die Nervenleitgeschwindigkeit weniger als ca. 2 m/s.

Im Sinne der Erfindung bezeichnet myelinisiert bevorzugt, dass die Nervenfaser von einer Markscheide umgeben ist. Diese wird auch als Myelinscheide bezeichnet. Die Funktion der Myelinscheide umfasst den Schutz der Neuronen vor fremden Aktionspotentialen und der schnelleren Erregungsausbreitung. Insbesondere wirkt die Myelinscheide isolierend, sodass die Membrankapazität verringert und Leckströme durch die Membran verhindert werden.

Begriffe wie im Wesentlichen, ungefähr, etwa, ca. etc. beschreiben bevorzugt einen Toleranzbereich von weniger als ± 40%, bevorzugt weniger als ± 20%, besonders bevorzugt weniger als ± 10 %, noch stärker bevorzugt weniger als ± 5% und insbesondere weniger als ± 1% und umfassen stets den exakten Wert. Ähnlich beschreibt bevorzugt Größen, die ungefähr gleich sind.

In einer weiteren bevorzugten Ausführungsform ist die Vorrichtung dadurch gekennzeichnet, dass der Schaltungsträger ein Material umfasst ausgewählt aus einer Gruppe umfassend faserverstärkter Kunststoff, Hartpapier, Teflon, Keramik, ein Metall und/oder ein Halbmetall umfassend Silizium, Kupfer, Aluminium, Eisen, Zink, Zinn, Nickel, Silber, Gold, deren Verbindungen und/oder deren Legierungen, wobei bevorzugt mehrere der aufgezählten Materialien für den Schaltungsträger ausgewählt sind.

Die genannten Materialien haben sich dahingehend als vorteilhaft erwiesen, dass hierdurch der Schaltungsträger besonders einfach und effizient bereitgestellt werden kann. Insbesondere sind die aufgezählten Materialien einfach zu bearbeiten, um sich an eine anatomische Form des Ohres individuell anzupassen. Weiterhin zeichnen sich die Materialien durch eine hohe Robustheit und Stabilität aus, wodurch die Vorrichtung vorteilhaft besonders langlebig und funktionstüchtig ist. Zudem ist es vorteilhaft, dass die genannten Materialien besonders günstig sind, sodass die Vorrichtung, insbesondere der Schaltungsträger, mit hoher Wirtschaftseffizienz bereitgestellt werden kann.

In bevorzugten Ausführungsformen weist der Schaltungsträger ein flexibles Trägermaterial auf Basis einer oder mehrere Kunststoffe. Bei den Kunststoffen bzw. Polymeren kann es sich bevorzugt um Elastomere handeln, insbesondere thermoplastische Elastomere. In bevorzugten Ausführungsformen kann das flexible Trägermaterial beispielsweise ausgewählt sein aus einer Gruppe umfassend Polyamid, PEEK; Styrol-Block-Copolymer und/oder Polyurethan.

Vorteilhaft ist es mit den derartigen Materialien möglich, einen flexiblen Schaltungsträger bereitzustellen, welche sich durch eine einfache Verformbarkeit oder Biegbarkeit auszeichnet. Durch die Flexibilität des Schaltungsträgers kann sich dieser durch Ausübung eines leichten Drucks oder Zuges derart verformen, dass sich der Schaltungsträger einer anatomisch individuellen Form des Ohres begünstigend anpasst und/oder anschmiegt. Durch elastisch ausgestaltete flexible Schaltungsträger kann zudem eine besonders stabiles Positionierung erreicht werden, sodass die elastische Rückstellkraft zusätzlichen Halt vermittelt.

In bevorzugten Ausführungsformen kann der Schaltungsträger ein FPCs (Flexible Printed Circuits), wobei die Flexibilität des Schaltungsträgers durch die Materialien und/oder das Herstellungsverfahren angepasst und/oder optimiert werden kann.

Der durchschnittliche Fachmann weiß, dass technische Merkmale, Definitionen und/oder Vorteile bevorzugter Ausführungsformen, welche für das erfindungsgemäßen Verfahren zur Herstellung der Vorrichtung zur transkutanen Aufbringung eines elektrischen Stimulationsreizes gleichermaßen für die Vorrichtung selbst gelten und umgekehrt.

In einem weiteren Aspekt betrifft die Erfindung ein Verfahren zur Herstellung der erfindungsgemäßen Vorrichtung, wobei die Fertigung des an die anatomische Form des Ohres angepassten Schaltungsträgers sowie der auf der Oberfläche des Schaltungsträgers aufgebrachten Elektroden, welche dazu konfiguriert sind, Bereiche des Ohres zu kontaktieren, auf Basis eines Modelles einer Oberfläche des Ohres und/oder eines Verlaufes von Nervenfasern und/oder Nervenbündel im Ohr durchgeführt wird.

Vorteilhafterweise wird ein auf die anatomische Form des Ohres optimal angepasster Schaltungsträger bereitgestellt, der langanhaltend und stabil am Ohr platziert werden kann. Darüber hinaus ist eine hohe Bequemlichkeit der Vorrichtung gegeben, indem insbesondere ein Modell einer Oberfläche des Ohres erstellt wird, sodass die Vorrichtung den Patienten weder mechanisch noch aus ästhetischen Gründen stört.

Die Aufnahme eines Modells zum Verlauf von Nervenfasern und/oder Nervenbündel ist besonders vorteilhaft, da eine besonders gezielte Aufbringung des elektrischen Stimulationsreiz garantiert wird. So werden vorteilhaft keine zu hohen und/oder nicht notwendigen Flächenbereiche des Ohres dem elektrischen Strom ausgesetzt. Weiterhin werden vorteilhaft Leckströme vermieden. Stattdessen werden vorteilhaft die Elektroden an den Stellen des Schaltungsträgers platziert, an denen sie auch mit geeigneten Stellen des Ohres kontaktiert werden, unterhalb dessen Hautoberfläche Nervenfasern und/oder Nervenbündel verlaufen, insbesondere des Vagusnervs. Somit wird die Wahrscheinlichkeit des Therapiererfolgs besonders erhöht, da der elektrische Strom mit enormer Präzision an Nervenfasern und/oder Nervenbündel übertragen wird.

Somit kann vorteilhafterweise die Vorrichtung eine ausgezeichnete Anpassung auf die anatomische Form des Ohres aufweisen als auch die Elektroden derart mit der Hautoberfläche in einer stabilen Berührung gebracht werden, sodass ein hoher Tragekomfort als auch eine besonders hohe Behandlungseffizienz gegeben ist.

Insbesondere ergibt sich die Behandlungseffizienz dadurch, dass der Schaltungsträger passgenau an eine anatomische Form des Ohres platziert werden kann, die Elektroden sich an geeigneten Positionen hinsichtlich des Verlaufs von Nervenfasern und/oder Nervenbündel, insbesondere des Vagusnervs, orientieren und ein optimaler Stimulationsreiz automatisch anhand einer Rückmeldung durch gemessene physiologische Parameter aufgebracht wird.

In einer weiteren bevorzugten Ausführungsform ist das Verfahren dadurch gekennzeichnet, dass folgende Schritte durchgeführt werden:
a) Abbildungsverfahren zur Aufnahme von Daten einer Oberfläche des Ohres und/oder eines Verlaufes von Nervenfasern und/oder Nervenbündel, bevorzugt von Nervenfasern und/oder Nervenbündel des Vagusnervs,
b) Erstellung eines Modells anhand der Daten über die Oberfläche des Ohres und/oder des Verlaufes von Nervenfasern und/oder Nervenbündel, bevorzugt von Nervenfasern und/oder Nervenbündel des Vagusnervs,
c) Übergabe des Modells an eine Fertigungsvorrichtung zur Fertigung des Schaltungsträgers mit Elektroden, wobei eine Oberfläche des Schaltungsträgers mindestens bereichsweise an eine anatomische Form des Ohres angepasst wird und die Elektroden derart auf der Oberfläche des Schaltungsträger aufgebracht werden, dass die Elektroden mit Bereichen des Ohres kontaktierbar sind.

Vorzugsweise werden die Verläufe von Nervenfasern und/oder Nervenbündel anhand der zugehörigen Blutgefäße in der Ohrmuschel ermittelt. Hierbei wird ausgenutzt, dass die Nervenfasern und/oder Nervenbündel mit dem Verlauf von Blutgefäßen korrelieren, oftmals liegen Nervenfasern neben Blutgefäßen vor bzw. sind mit diesen verflochten. Beispielsweise können Nervenfasern und Blutgefäße einen Abstand von ca. 200 µm zueinander haben. Um Informationen über den Verlauf, insbesondere hinsichtlich der Positionierung, von Nervenfasern und/oder Nervenbahnen zu erhalten, wird bevorzugt eine Transilluminationsmethode eingesetzt, um diese zu visualisieren. Die dabei zu erkennenden Blutgefäße bilden Regionen, die die höchste Wahrscheinlichkeit aufweisen, Nervenfasern und/oder Nervenbündel zu beinhalten. Dazu kann es bevorzugt sein, das Ohr mit grünem Licht zu beleuchten, da insbesondere Blutgefäße für grünes Licht weniger transparent sind als das umgebende Gewebe. Die visualisierte Lage der Gefäße zeigt die wahrscheinlichsten Regionen der Nerven an, die für eine individuelle Kontaktierung mit den Elektroden geeignet sind. Vorzugsweise wird die Lage und/oder der Verlauf von Nervenfasern und/oder Nervenbündel durch ein Bild aufgenommen.

Transillumination bezeichnet bevorzugt allgemein ein Verfahren, welches zur Beleuchtung von Proben und/oder Abschnitten des Körpers eingesetzt werden kann, insbesondere im Zusammenhang mit Bildgebungsverfahren.

Zur Aufnahme einer anatomischen Form des Ohres wird bevorzugt ein dreidimensionales Bild des Ohres aufgenommen. Bevorzugt wird ein dreidimensionales Bild mit einer Auflösung erfasst, welche ein besonders genaues Abbild der individuellen Gestaltung und Form eines Ohres gewährleistet. Insbesondere sollen vorzugsweise eine Topologie einer anatomischen Form des Ohres im Submillimeterbereich hinreichend präzise aufgelöst werden, um eine entsprechende Gestaltung der Oberfläche des Schaltungsträgers vorzugeben. Durch die Aufnahme eines dreidimensionale Bildes eines Teilbereiches des Ohres, beispielsweise einer Ohrmuschel oder Teilen davon, kann zudem ein besonders präzises Modell des entsprechenden Abschnittes erstellt werden, welches weiterverarbeitet werden kann.

Vorzugsweise werden auf Basis eines Abbildungsverfahrens einer Oberfläche des Ohres und/oder des Verlaufs der Nervenfasern und/oder Nervenbündel Daten erstellt, die diese wiedergeben. Bevorzugt wird auf Basis der Daten ein Modell erstellt, das eine anatomische Form des Ohres, bevorzugt einen Abschnitt der Oberfläche des Außenohrs und/oder die Position der Nervenfasern und/oder Nervenbündel unterhalb der Oberfläche enthält und berücksichtigt. Somit wird vorteilhaft eine optimale Anpassung des Schaltungsträger am Ohr des Patienten und eine genaueste Platzierung der Elektroden im Hinblick auf den Verlauf von Nervenfasern und/oder Nervenbündeln ermöglicht. Das Außenohr umfasst die Ohrmuschel und den äußeren Gehörgang. Somit kann durch die bevorzugt zumindest abschnittsweise Aufnahme des Außenohr ein Schaltungsträger bereitgestellt werden, der beispielsweise wie ein Kopfhörer angepasst auf die anatomische Form in oder an dem äußeren Gehörgang eingebracht werden kann. In weiteren bevorzugten Ausführungsformen kann der Schaltungsträger an Bereichen der Ohrmuschel angebracht werden. In anderen bevorzugten Ausführungsformen kann der Schaltungsträger an mehreren Bereichen der Ohrmuschel aufliegen und dabei die Ausgestaltung eines Hörgerätes aufweisen. Dabei werden bevorzugt in den Ausführungsformen die anatomischen Strukturen und/oder Konturen des Außenohrs berücksichtigt, sodass, unabhängig von den Ausmaßen des Schaltungsträgers, dieser kongruent angebracht werden kann.

Das Model wird auf Basis der Abbildungsverfahren zur Aufnahme von Daten einer Oberfläche des Ohres und/oder eines Verlaufes von Nervenfasern und/oder Nervenbündel, bevorzugt des Vagusnervs, gebildet.

Das Modell bezeichnet bevorzugt ein mittels eines computerimplementieren Verfahrens generierte digitale Repräsentation eines Teilbereiches des Ohres, welches auf Messdaten eines Abbildungsverfahrens einer Oberfläche des Ohres und/oder des Verlaufs der Nervenfasern und/oder Nervenbündeln basiert, wobei zusätzliche Verarbeitungsschritte vorgesehen sein können. Beispielsweise kann in dem Modell eine Interpolation vorgenommen werden, um Messtoleranzen auszugleichen, mittels nummerischer Methode eine Genauigkeit der Bestimmung des Verlaufes von Nervenfasern und/oder Nervenbündeln zu erhöhen und/oder eine Auflösung einer dreidimensional erfassten Oberfläche an anschließende Fertigungsverfahren anzupassen (bspw. durch geeignete Glättungsverfahren). Das erstellte Modell kann auf einem Abbildungsverfahren eines individuellen Ohres basieren und mithin eine individuelle Anpassung der Vorrichtung an einen Träger ermöglichen. Ebenso ist es möglich, dass in das Model Daten zu unterschiedlichen Ohren aufgenommen wurden, um eine Mittelung zu erreichen.

Bevorzugt wird das Modell an eine Fertigungsvorrichtung übergeben. Vorteilhaft wird bereits während der Fertigung der Vorrichtung die Anpassung auf die anatomische Form des Ohres berücksichtigt, sodass ein an das Ohr passgenauer Schaltungsträger erstellt werden kann. Gleichzeitig können vorteilhaft die Elektroden derart auf den Schaltungsträger platziert werden, um eine optimale Kontaktierung an den Stellen der Hautoberfläche zu ermöglichen, unter denen geeignete und/oder ein hoher Anteil an Nervenfasern und/oder Nervenbündel verläuft, bevorzugt des Vagusnervs.

Das Model liegt vorzugsweise in einem Dateiformat vor, das die Fertigungsvorrichtung verarbeiten kann. Vorzugsweise erstellt die Fertigungsvorrichtung anhand des eingegeben Modells den Schaltungsträger. Bevorzugte Dateiformate sind ausgewählt aus einer Gruppe umfassend CAD-Formate (engl. computer aided desing) umfassend IGES (Initial Graphics Exchange Specification)- und/oder STP-Formate, Mesh-Formate umfassend STL-, X3D- und/oder COLLADA- (engl. COLLAborative Design Activity) Formate und/oder Farb-Formate umfassend VRML- (engl. Virtual Reality Modeling Language), OBJ-, PLY- (PoLYgon-File) und/oder AMF- (engl. Additive Manufacturing File Format) Formate.

In einer weiteren bevorzugten Ausführungsform ist das Verfahren dadurch gekennzeichnet, dass die Fertigung des Schaltungsträgers mit Elektroden ein additives Fertigungsverfahren umfasst.

Vorteilhafterweise kann der Schaltungsträger besonders einfach und effizient bereitgestellt werden. Besonders vorteilhaft ist der Umstand, dass bereits während der Erstellung des Schaltungsträgers Komponenten wie beispielsweise die Elektroden und/oder Leiterbahnen angebracht werden können, um schließlich den elektrischen Stimulationsreiz aufzubringen. So müssen die Komponenten nicht erst nachträglich am Schaltungsträger aufgebracht werden. Stattdessen kann der Schaltungsträger mit darauf vorliegenden Komponenten wie Elektroden und/oder Leiterbahnen besonders prozesseffizient hergestellt werden.

In bevorzugten Ausführungsformen werden weitere Komponenten wie der mindestens eine erste Sensor und/oder der mindestens eine zweite Sensor ebenfalls durch das additive

Fertigungsverfahren im Schaltungsträger eingesetzt bzw. positioniert. Besonders bevorzugt liegt nach der additiven Fertigung eine elektrische Verbindung zwischen den Elektroden und der Steuerungseinheit vor, vorzugsweise auch zwischen dem ersten und/oder dem zweiten Sensor und der Steuerungseinheit. In weiteren bevorzugten Ausführungsformen wird durch das additive Fertigungsverfahren ein Schaltungsträger bereitgestellt. Komponenten, wie beispielsweise der mindestens eine erste Sensor und/oder der mindestens eine zweite Sensor, können bevorzugt nach einer Bereitstellung des Schaltungsträger daran integriert werden. Insbesondere weist der Schaltungsträger nach seiner Bereitstellung bevorzugt durch ein additives Fertigungsverfahren Isoliermaterial, Leiterbahnen und/oder Elektroden auf. Vorzugsweise können daraufhin Komponenten wie der mindestens eine erste Sensor, der mindestens eine zweite Sensor und/oder die Steuerungseinheit mit herkömmlichen Methoden der Halbleiter-, Mikrosystem-, Verbindungs- und/oder Aufbau- und Verbindungstechnik an dem Schaltungsträger angebracht werden.

Besonders bevorzugt wird als additives Fertigungsverfahren ein multimaterielles additives Fertigungsverfahren eingesetzt. Vorteilhafterweise lässt sich durch ein multimaterielles additives Fertigungsverfahren die Anpassung auf eine anatomische Form des Ohres besonders einfach gestalten. Weiterhin lassen sich vorteilhaft Komponenten wie die Elektroden und/oder Leiterbahnen in einem Prozessablauf integrieren, verbauen und/oder kombinieren. Insbesondere werden vorteilhaft in einem Ablauf mehrere, für die jeweiligen Komponenten geeignete, Materialien zur Verfügung gestellt, um die Vorrichtung und/oder die Komponenten in einer einzigen Abfolge bereitzustellen. So werden bevorzugt elektrisch leitende Materialien eingesetzt, um Leiterbahnen bereitzustellen auf dem Substrat. Das Substrat kann als Basiskörper des Schaltungsträger aufgefasst werden, wobei das Substrat bevorzugt elektrisch isolierendes Material aufweist. Vorzugsweise können auch Elektroden durch das bevorzugt eingesetzte multimaterielle additive Fertigungsverfahren auf dem Substrat bereitgestellt werden, beispielsweise als metallische Abschnitte. Vorzugsweise kann die Flexibilität des Schaltungsträgers durch eine flexible Ausgestaltung des Substrats ermöglicht werden, indem ein oder mehrere geeignete Materialien, wie beispielsweise Kunststoffe, eingesetzt werden. Durch die Flexibilität ist vorteilhaft eine leichte Verformbarkeit des Schaltungsträgers gegeben, um eine besonders präzise Anpassung an eine anatomische Form des Ohres zu ermöglichen. Bevorzugte Komponenten der erfindungsgemäßen Vorrichtung wie beispielsweise die Steuerungseinheit, der mindestens eine erste Sensor und/oder der mindestens eine zweite Sensor können mit in der Halbleitertechnologie bekannten Verfahren über die Leiterbahnen miteinander verbunden und auf dem Schaltungsträger angebracht werden. Der Schaltungsträger kann somit vorzugsweise als Halterung für Komponenten wie Leiterbahnen, der mindestens eine erste Sensor, Elektroden etc. aufgefasst werden, der gleichzeitig am Ohr eines Probanden auf- oder eingebracht werden kann.

Ein additives Fertigungsverfahren (synonym auch 3D-Druck) bezeichnet ein Verfahren, bei dem das Bauteil, im erfindungsgemäßen Kontext die Vorrichtung, insbesondere der Schaltungsträger, Schicht für Schicht aufgebaut wird. Dazu wird das dafür eingesetzte Material aus einer Düse extrudiert und auf eine Plattform aufgetragen. Meist ist die Düse an einem Druckkopf verbaut, der dazu konfiguriert ist, ein-, zwei und/oder dreidimensionale Bewegungen auszuführen. Insbesondere ist es bei additiven Fertigungsverfahren nicht notwendig, Material abzutragen, beispielsweise durch fräsende Bearbeitungen. Vorteilhaft ergibt sich durch additive Fertigungsverfahren eine enorme Flexibilität und Gestaltungsfreiheit. Besonders vorteilhaft ist auch, dass die Vorrichtung und/oder Komponenten der Vorrichtung in einer Massenanfertigung durch Serienproduktion hergestellt werden können, was die Wirtschaftlichkeit besonders erhöht.

In multimateriellen additiven Fertigungsverfahren werden mehrere Materialien eingesetzt, insbesondere innerhalb eines Prozessablaufes. So kann es beispielsweise bevorzugt sein, dass mehrere Druckköpfe eingesetzt werden, aus deren Düsen verschiedene Materialien extrudiert werden. So kann aus einer Düse metallisches Material extrudiert werden, beispielsweise für die Elektroden und/oder für die Leiterbahnen. Für das Substrat des Schaltungsträger selbst kann beispielsweise elektrisch isolierendes Material eingesetzt werden.

Vorteilhaft können auch in einem multimateriellen, additiven Fertigungsverfahren Materialien eingesetzt werden, deren Eigenschaften kontinuierlich entlang einer Raumrichtung, mehrerer Raumrichtungen und/oder einer geometrischen Form ausgeprägt sind. Eine solche Veränderung lässt sich erzielen, wenn die Materialzusammensetzung und/oder die Prozessparameter im additiven Fertigungsverfahren modifiziert werden. Vorteilhaft lassen sich die Materialeigenschaften gezielt einstellen. Beispielsweise kann der Schaltungsträger einen fließenden hart-weich-Verlauf haben, um an Materialübergängen vorteilhaft eine sehr geringe Rissanfälligkeit aufzuweisen.

### LITERATURVERZEICHNIS

Ekmek 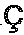 i, Hakan, Hülagu, Kaptan. "Vagus nerve stimulation." *Open access Macedonian journal of medical sciences* 5.3 (2017): 391.
Kaniusas, Eugenijus, et al. "Current directions in the auricular vagus nerve stimulation II-an engineering perspective." Frontiers in neuroscience 13 (2019): 772.

## Patentansprüche

1. Vorrichtung zur transkutanen Aufbringung eines elektrischen Stimulationsreizes auf ein Ohr umfassend einen Schaltungsträger, mindestens zwei Elektroden sowie eine Steuerungseinheit,
wobei eine Oberfläche des Schaltungsträgers an eine anatomische Form des Ohres angepasst ist und die Elektroden derart auf der Oberfläche des Schaltungsträgers aufgebracht vorliegen, dass die Elektroden mit Bereichen des Ohres kontaktiert sind, wobei die Steuerungseinheit dazu konfiguriert ist, anhand von Stimulationsparametern ein elektrisches Stimulationssignal an den Elektroden zu erzeugen, wobei die Vorrichtung mindestens einen ersten Sensor zur Erkennung mindestens eines physiologischen Parameter umfasst und die Steuerungseinheit dazu konfiguriert ist, anhand einer Messung des physiologischen Parameters die Stimulationsparameter anzupassen, wobei die Vorrichtung mindestens drei oder mehr Elektroden aufweist,
**dadurch gekennzeichnet, dass**
die drei oder mehr Elektroden als Array angeordnet vorliegen, wobei mittels der Steuerungseinheit die Elektroden einzeln steuerbar sind
und die Steuerungseinheit dazu konfiguriert ist, anhand einer Auswertung des gemessenen physiologischen Parameters eine Auswahl von Elektroden zu ermitteln, welche besonders geeignet sind, durch einen elektrischen Stimulationsreiz eine Veränderung des physiologischen Parameters zu bewirken.

2. Vorrichtung nach dem vorherigen Anspruch
**dadurch gekennzeichnet, dass**
eine Anpassung der Stimulationsparameter anhand der gemessen physiologischen Parameter in einem geschlossenen Regelkreis erfolgt, wobei bevorzugt die Anpassung der Stimulationsparameter zur Führung des physiologischen Parameters auf einen vorgegebenen Zielwert erfolgt.

3. Vorrichtung nach einem oder mehreren der vorherigen Ansprüche
**dadurch gekennzeichnet, dass**
die an eine anatomische Form des Ohres angepasste Oberfläche des Schaltungsträgers und/oder die Positionierung der Elektroden auf der Oberfläche des Schaltungsträgers zur Kontaktierung von Bereichen des Ohres auf Daten eines Abbildungsverfahrens eines Bereiches einer Oberfläche des Ohres und/oder eines Verlaufes von Nervenfasern und/oder Nervenbündel, bevorzugt Nervenfasern und/oder Nervenbündel des Vagusnervs, basiert.

4. Vorrichtung nach einem oder mehreren der vorherigen Ansprüche
**dadurch gekennzeichnet, dass**
die Vorrichtung in den Gehörgang einsetzbar ist, wobei eine Oberfläche des Schaltungsträgers an einen Bereich des Gehörgangs angepasst ist und die Elektroden bevorzugt einen Bereich der Concha und/oder des äußeren Gehörgangs des Ohres kontaktieren.

5. Vorrichtung nach einem oder mehreren der vorherigen Ansprüche
**dadurch gekennzeichnet, dass**
die Vorrichtung in die Ohrmuschel einsetzbar ist und eine Oberfläche des Schaltungsträgers an einen Bereich der Ohrmuschel angepasst ist, wobei die Elektroden bevorzugt einen Bereich der Concha, bevorzugt Cymba Concha oder Cavum Concha, des Tragus, der Fossa triangularis, der Scapha, der Anthelix und/oder des Antitragus der Ohrmuschel kontaktieren.

6. Vorrichtung nach einem oder mehreren der vorherigen Ansprüche
**dadurch gekennzeichnet, dass**
die Vorrichtung mindestens 5, 10, 20, 50, 100 oder mehr Elektroden aufweist.

7. Vorrichtung nach einem oder mehreren der vorherigen Ansprüche
**dadurch gekennzeichnet, dass**
die Steuerungseinheit dazu konfiguriert ist, mit Algorithmen der künstlichen Intelligenz, bevorzugt mit Machine-Learning Algorithmen umfassend Supervised Learning, Unsupervised Learning und/oder Reinforcement Learning, eine Auswahl von Elektroden zu ermitteln, welche besonders geeignet sind, durch einen elektrischen Stimulationsreiz eine Veränderung des physiologischen Parameters zu bewirken.

8. Vorrichtung nach einem oder mehreren der vorherigen Ansprüche
**dadurch gekennzeichnet, dass**
der mindestens eine erste Sensor ausgewählt ist aus einer Gruppe umfassend ein Temperatursensor, ein Bewegungssensor, ein Pulssensor, ein Blutdrucksensor, ein Blutflusssensor, einen Pulsoximetriesensor und/oder ein pH-Sensor.

9. Vorrichtung nach einem oder mehreren der vorherigen Ansprüche
**dadurch gekennzeichnet, dass**
die Vorrichtung mindestens einen zweiten Sensor umfasst, wobei der mindestens eine zweite Sensor dazu konfiguriert ist, Umweltparameter zu messen, wobei bevorzugt die Umweltparameter ausgewählt sind aus einer Gruppe umfassend eine Umgebungstemperatur, einen Luftdruck und/oder eine Luftfeuchtigkeit, wobei die Steuerungseinheit bevorzugt dazu konfiguriert ist, eine Messung von Umweltparametern für die Anpassung der Stimulationsparameter für den elektrischen Stimulationsreiz zu berücksichtigen.

10. Vorrichtung nach einem oder mehreren der vorherigen Ansprüche
**dadurch gekennzeichnet, dass**
die Vorrichtung eine Kommunikationseinheit zur Verbindung mit einer externen Datenverarbeitungseinheit und/oder einem Eingabemodul umfasst.

11. Vorrichtung nach einem oder mehreren der vorherigen Ansprüche
**dadurch gekennzeichnet, dass**
das elektrische Signal durch einen Gleichstrom oder einen Wechselstrom bereitgestellt wird, wobei bevorzugt im Falle eines Wechselstroms eine Frequenz zwischen 0,1 - 100 Hz vorliegt, wobei bei Wechselstrom bevorzugt eine konstante Impulsfolge, eine unterbrochene Impulsfolge und/oder ein Rechtecksignal eingesetzt wird.

12. Verfahren zur Herstellung einer Vorrichtung gemäß einem oder mehreren der vorherigen Ansprüche
wobei
die Fertigung des an die anatomische Form des Ohres angepassten Schaltungsträgers sowie der auf der Oberfläche des Schaltungsträgers aufgebrachten Elektroden, welche dazu konfiguriert sind, Bereiche des Ohres zu kontaktieren, auf Basis eines Modelles einer Oberfläche des Ohres und/oder eines Verlaufes von Nervenfasern und/oder Nervenbündel im Ohr durchgeführt wird.

13. Verfahren nach dem vorherigen Anspruch
**dadurch gekennzeichnet, dass**
folgende Schritte durchgeführt werden:
a) Abbildungsverfahren zur Aufnahme von Daten einer Oberfläche des Ohres und/oder eines Verlaufes von Nervenfasern und/oder Nervenbündel, bevorzugt von Nervenfasern und/oder Nervenbündel des Vagusnervs,
b) Erstellung eines Modells anhand der Daten über die Oberfläche des Ohres und/oder des Verlaufes von Nervenfasern und/oder Nervenbündel, bevorzugt von Nervenfasern und/oder Nervenbündel des Vagusnervs,
c) Übergabe des Modells an eine Fertigungsvorrichtung zur Fertigung des Schaltungsträgers mit Elektroden, wobei eine Oberfläche des Schaltungsträgers mindestens bereichsweise an eine anatomische Form des Ohres angepasst wird und die Elektroden derart auf der Oberfläche des Schaltungsträger aufgebracht werden, dass die Elektroden mit Bereichen des Ohres kontaktierbar sind.

14. Verfahren nach Anspruch 12 oder 13
**dadurch gekennzeichnet, dass**
die Fertigung des Schaltungsträgers mit Elektroden ein additives Fertigungsverfahren umfasst.

## Claims

1. A device for transcutaneously applying an electrical stimulation stimulus to an ear, comprising a circuit carrier, at least two electrodes, and a control unit,
wherein a surface of the circuit carrier is adapted to an anatomical shape of the ear and the electrodes are provided on the surface of the circuit carrier in such a way that the electrodes are in contact with areas of the ear,
wherein the control unit is configured to generate an electrical stimulation signal at the electrodes based on stimulation parameters, wherein the device comprises at least one first sensor for detecting at least one physiological parameter and the control unit is configured to adjust the stimulation parameters based on a measurement of the physiological parameter,
wherein the device has at least three or more electrodes,
**characterized in that**
the three or more electrodes are arranged in an array, wherein the electrodes can be controlled individually by means of the control unit,
and the control unit is configured to determine, on the basis of an evaluation of the measured physiological parameter, a selection of electrodes that are particularly suitable for causing a change in the physiological parameter by means of an electrical stimulation stimulus.

2. The device of the preceding claim,
**characterized in that**
an adjustment of the stimulation parameters is carried out on the basis of the measured physiological parameters in a closed loop, preferably wherein the adjustment of the stimulation parameters is carried out to guide the physiological parameter to a predetermined target value.

3. The device of any one or more of the preceding claims, **characterized in that**
the surface of the circuit carrier adapted to an anatomical shape of the ear and/or the positioning of the electrodes on the surface of the circuit carrier for contacting areas of the ear is based on data from a method for imaging an area of a surface of the ear and/or a course of nerve fibers and/or nerve bundles, preferably nerve fibers and/or nerve bundles of the vagus nerve.

4. The device of any one or more of the preceding claims, **characterized in that**
the device can be inserted into the ear canal, with a surface of the circuit carrier being adapted to an area of the ear canal and the electrodes preferably contacting an area of the concha and/or the external ear canal.

5. The device of any one or more of the preceding claims, **characterized in that**
the device can be inserted into the auricle and a surface of the circuit carrier is adapted to an area of the auricle, wherein the electrodes preferably contact an area of the concha, preferentially cymba concha or cavum concha, the tragus, the fossa triangularis, the scapha, the anthelix, and/or the antitragus of the auricle.

6. The device of any one or more of the preceding claims, **characterized in that**
the device has at least 5, 10, 20, 50, 100 or more electrodes.

7. The device of any one or more of the preceding claims, **characterized in that**
the control unit is configured to use artificial intelligence algorithms, preferably machine learning algorithms, comprising supervised learning, unsupervised learning, and/or reinforcement learning, to determine a selection of electrodes that are particularly suitable for causing a change in the physiological parameter by means of an electrical stimulation stimulus.

8. The device of any one or more of the preceding claims, **characterized in that**
the at least one first sensor is selected from a group comprising a temperature sensor, a motion sensor, a pulse sensor, a blood pressure sensor, a blood flow sensor, a pulse oximetry sensor, and/or a pH sensor.

9. The device of any one or more of the preceding claims, **characterized in that**
the device comprises at least one second sensor, wherein the at least one second sensor is configured to measure environmental parameters, wherein the environmental parameters are preferably selected from a group comprising an ambient temperature, an air pressure, and/or an air humidity, wherein the control unit is preferably configured to take into account a measurement of environmental parameters for adapting the stimulation parameters for the electrical stimulation stimulus.

10. The device of any one or more of the preceding claims, **characterized in that**
the device comprises a communication unit for connection to an external data processing unit and/or an input module.

11. The device of any one or more of the preceding claims, **characterized in that**
the electrical signal is provided by means of a direct current or an alternating current, preferably with a frequency between 0.1 and 100 Hz in the case of an alternating current, and preferably using a constant pulse sequence, an interrupted pulse sequence, and/or a square-wave signal in the case of an alternating current.

12. A method of manufacturing a device of any one or more of the preceding claims,
wherein
the manufacture of the circuit carrier, which is adapted to the anatomical shape of the ear, and of the electrodes applied to the surface of the circuit carrier, which are configured to contact areas of the ear, is performed on the basis of a model of a surface of the ear and/or a course of nerve fibers and/or nerve bundles in the ear.

13. The method of the preceding claim,
**characterized in that**
the following steps are performed:
a) an imaging method for recording data from a surface of the ear and/or a course of nerve fibers and/or nerve bundles, preferably of nerve fibers and/or nerve bundles of the vagus nerve,
b) creating a model based on data about the surface of the ear and/or the course of nerve fibers and/or nerve bundles, preferably of nerve fibers and/or nerve bundles of the vagus nerve,
c) transferring the model to a manufacturing device for manufacturing the circuit carrier with electrodes, wherein a surface of the circuit carrier is adapted at least in areas to an anatomical shape of the ear and the electrodes are applied to the surface of the circuit carrier in such a way that the electrodes can be brought into contact with areas of the ear.

14. The method of claim 12 or 13,
**characterized in that**
the manufacture of the circuit carrier with electrodes comprises an additive manufacturing process.

## Revendications

1. Dispositif d'application transcutanée d'une excitation de stimulation électrique sur une oreille comprenant un support de circuit, au moins deux électrodes ainsi qu'une unité de commande,
dans lequel une surface du support de circuit est adaptée à une forme anatomique de l'oreille et les électrodes sont appliquées sur la surface du support de circuit de sorte que les électrodes sont en contact avec des régions de l'oreille,
dans lequel l'unité de commande est configurée pour générer un signal de stimulation électrique sur les électrodes sur la base de paramètres de stimulation, dans lequel le dispositif comprend au moins un premier capteur pour détecter au moins un paramètre physiologique et l'unité de commande est configurée pour ajuster les paramètres de stimulation sur la base d'une mesure du paramètre physiologique,
dans lequel le dispositif comprend au moins trois électrodes ou plus,
**caractérisé en ce que**
les trois électrodes ou plus sont disposées sous forme de réseau, dans lequel les électrodes peuvent être commandées individuellement au moyen de l'unité de commande et l'unité de commande est configurée pour déterminer, sur la base d'une évaluation du paramètre physiologique mesuré, une sélection d'électrodes particulièrement aptes à provoquer une modification du paramètre physiologique par une excitation de stimulation électrique.

2. Dispositif selon la revendication précédente **caractérisé en ce que**
un ajustement des paramètres de stimulation à l'aide des paramètres physiologiques mesurés s'effectue dans un circuit de régulation fermé, dans lequel, de préférence, l'ajustement des paramètres de stimulation s'effectue pour guider le paramètre physiologique à une valeur cible prédéterminée.

3. Dispositif selon l'une ou plusieurs des revendications précédentes
**caractérisé en ce que**
la surface du support de circuit adaptée à une forme anatomique de l'oreille et/ou le positionnement des électrodes sur la surface du support de circuit pour la mise en contact de régions de l'oreille sont basés sur des données d'un procédé d'imagerie d'une région d'une surface de l'oreille et/ou d'un tracé de fibres nerveuses et/ou de faisceaux nerveux, de préférence de fibres nerveuses et/ou de faisceaux nerveux du nerf vague.

4. Dispositif selon l'une ou plusieurs des revendications précédentes
**caractérisé en ce que**
le dispositif peut être inséré dans le conduit auditif, dans lequel une surface du support de circuit est adaptée à une région du conduit auditif et les électrodes sont de préférence en contact avec une région de la conque et/ou du conduit auditif externe de l'oreille.

5. Dispositif selon l'une ou plusieurs des revendications précédentes
**caractérisé en ce que**
le dispositif peut être inséré dans le pavillon de l'oreille et une surface du support de circuit est adaptée à une région du pavillon de l'oreille, dans lequel les électrodes recouvrent de préférence une région de la conque, de préférence de la conque cymba ou de la conque cavum, du tragus, du triangulaire fosse, le scapha, l'antihélix et/ou l'antitragus du pavillon de l'oreille.

6. Dispositif selon l'une ou plusieurs des revendications précédentes
**caractérisé en ce que**
le dispositif comprend au moins 5, 10, 20, 50, 100 électrodes ou plus.

7. Dispositif selon l'une ou plusieurs des revendications précédentes
**caractérisé en ce que**
l'unité de commande est configurée pour utiliser des algorithmes d'intelligence artificielle, de préférence des algorithmes d'apprentissage automatique comprenant un apprentissage supervisé, un apprentissage non supervisé et/ou un apprentissage par renforcement, pour déterminer une sélection d'électrodes qui sont particulièrement adaptées pour provoquer une modification du paramètre physiologique par l'intermédiaire d'une excitation de stimulation électrique.

8. Dispositif selon l'une ou plusieurs des revendications précédentes
**caractérisé en ce que**
au moins un premier capteur est sélectionné dans un groupe comprenant un capteur de température, un capteur de mouvement, un capteur de pouls, un capteur de pression sanguine, un capteur de flux sanguin, un capteur d'oxymétrie de pouls et/ou un capteur de pH.

9. Dispositif selon l'une ou plusieurs des revendications précédentes
**caractérisé en ce que**
le dispositif comprend au moins un second capteur, dans lequel l'au moins un second capteur est configuré pour mesurer des paramètres environnementaux, de préférence dans lequel les paramètres environnementaux sont sélectionnés dans un groupe comprenant une température ambiante, une pression atmosphérique et/ou une humidité atmosphérique, dans lequel, de préférence, l'unité de commande est configurée pour prendre en compte une mesure de paramètres environnementaux pour l'ajustement des paramètres de stimulation de l'excitation de stimulation électrique.

10. Dispositif selon l'une ou plusieurs des revendications précédentes
**caractérisé en ce que**
le dispositif comprend une unité de communication destinée à être connectée à une unité de traitement de données externe et/ou à un module d'entrée.

11. Dispositif selon l'une ou plusieurs des revendications précédentes
**caractérisé en ce que**
le signal électrique est fourni par un courant continu ou un courant alternatif, dans lequel, de préférence dans le cas d'un courant alternatif, il existe une fréquence comprise entre 0,1 et 100 Hz, dans lequel, dans le cas d'un courant alternatif, on utilise de préférence une séquence d'impulsions constante, une séquence d'impulsions interrompue et/ou un signal rectangulaire.

12. Procédé de fabrication d'un dispositif selon l'une ou plusieurs des revendications précédentes
dans lequel
la fabrication du support de circuit adapté à la forme anatomique de l'oreille ainsi que des électrodes placées sur la surface du support de circuit, lesquelles électrodes sont configurées pour entrer en contact avec des régions de l'oreille, est réalisée sur la base d'un modèle d'une surface de l'oreille et/ou d'un tracé de fibres nerveuses et/ou de faisceaux nerveux dans l'oreille.

13. Procédé selon la revendication précédente
**caractérisé en ce que**
les étapes suivantes sont effectuées :
a) procédé d'imagerie pour l'enregistrement de données d'une surface de l'oreille et/ou d'un tracé de fibres nerveuses et/ou de faisceaux nerveux, de préférence de fibres nerveuses et/ou de faisceaux nerveux du nerf vague,
b) création d'un modèle basé sur les données sur la surface de l'oreille et/ou le trajet des fibres nerveuses et/ou des faisceaux nerveux, de préférence des fibres nerveuses et/ou des faisceaux nerveux du nerf vague,
c) transfert du modèle à un dispositif de fabrication pour la fabrication du support de circuit avec des électrodes, dans lequel une surface du support de circuit est adaptée au moins par endroits à une forme anatomique de l'oreille et les électrodes sont appliquées sur la surface du support de circuit de sorte que les électrodes peuvent être mises en contact avec des régions de l'oreille.

14. Procédé selon la revendication 12 ou 13,
**caractérisé en ce que**
la fabrication du support de circuit avec des électrodes comprend un procédé de fabrication additif.
